(19) Europäisches Patentamt
European Patent Office
Office européen des brevets

(11) EP 4 349 822 A1

(12) **EUROPEAN PATENT APPLICATION**
published in accordance with Art. 153(4) EPC

(43) Date of publication:
**10.04.2024 Bulletin 2024/15**

(21) Application number: **22810558.1**

(22) Date of filing: **24.05.2022**

(51) International Patent Classification (IPC):
***C07D 317/50*** *(2006.01)* ***A61K 31/36*** *(2006.01)*
***A61P 25/24*** *(2006.01)*

(52) Cooperative Patent Classification (CPC):
**A61K 31/36; A61P 25/22; A61P 25/24; C07D 317/50; C07D 317/60**

(86) International application number:
**PCT/CN2022/094753**

(87) International publication number:
**WO 2022/247834 (01.12.2022 Gazette 2022/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.05.2021 CN 202110606431**

(71) Applicant: **Tasly Pharmaceutical Group Co., Ltd.**
**Tianjin 300410 (CN)**

(72) Inventors:
- **MA, Xiaohui**
  **Tianjin 300410 (CN)**
- **GAO, Xucong**
  **Tianjin 300410 (CN)**
- **WANG, Xiangyang**
  **Tianjin 300410 (CN)**
- **LI, Xiaoqing**
  **Tianjin 300410 (CN)**
- **LIU, Rui**
  **Tianjin 300410 (CN)**
- **ZHOU, Shuiping**
  **Tianjin 300410 (CN)**

(74) Representative: **HGF**
**HGF Limited**
**1 City Walk**
**Leeds LS11 9DX (GB)**

# (54) ANTIDEPRESSANT AND ANXIOLYTIC SUBSTITUTED CINNAMAMIDE COMPOUND

(57) The present application relates to an antidepressant and anxiolytic substituted cinnamamide compound, i.e., compound M2. The present application also provides a pharmaceutical composition containing compound M2 or a solvate thereof or a pharmaceutically acceptable salt thereof, and an antidepressant, anxiolytic, or antidepressant and anxiolytic application.

EP 4 349 822 A1

## Description

Related application

The present application claims priority of Chinese Invention Patent Application No. 202110606431.2, filed on May 28, 2021 and titled "Novel antidepressant and anxiolytic compound", the entire contents of which are incorporated herein by reference.

Technical Field

The present application relates to, but is not limited to, a pharmaceutical compound, in particular to a novel antidepressant and anxiolytic substituted cinnamamide compound.

Background

Depression and anxiety are the main diseases that harm human mental health. With the accelerated pace of modern life, the population with the diseases is expanding year by year. Recently, according to statistics, the incidence of various types of depression in China has reached 26 million, with teenagers accounting for a larger proportion. Therefore, research and development of antidepressant and anxiolytic drugs have important economic and social benefits.

Numerous studies suggest that changes in central monoamine neurotransmitters, dopamine, cholinergic changes, changes in corresponding receptor functions, as well as neuroendocrine dysfunction may play an important role in the occurrence and development of the diseases. So far, the therapeutic principle should focus on adjusting the content of hypothalamic monoamine neurotransmitters and their receptor functions and restoring normal neuroendocrine.

At present, the major means for treating depression and anxiety is still taking drugs. At present, antidepressant and anxiolytic drugs in clinic mainly include chemical drugs such as tricyclics, benzodiazepines, 5-hydroxytryptamine reuptake inhibitors, and monoamine oxidase inhibitors. They have certain therapeutic effect on depression and anxiety, but they also have relatively significant toxic side effect: monoamine oxidase inhibitors have some toxic side effect, because they lead to toxic liver damage due to selectivity and irreversible inhibition of enzymes. Commonly used tricyclic drugs include doxepin, amitriptyline, clomipramine, etc. Although these drugs have a good effect on endogenous depression, especially depressed mood, diminished interest and pessimism, for which a therapeutic effect of 80% or more can be achieved, they have many adverse reactions due to their significant toxicity to heart. Selective 5-HT reuptake inhibitors (SSRI) are novel antidepressant and anxiolytic drugs that emerged in the late 1980s. They have become first-line drugs commonly used in Europe and the United States, because they can significantly reduce the adverse reactions of other receptors while maintaining classical antidepressant and anxiolytic properties. Commonly used drugs include fluoxetine, paroxetine, sertraline, citalopram, fluvoxamine, etc. Due to gastrointestinal absorption and liver metabolism, these drugs still have the problem of gastrointestinal dysfunction, and also lead to sexual dysfunction in some patients, which affects the long-term use for treatment to a certain extent. Moreover, conventional antidepressant and anxiolytic drugs take effect slowly (6-8 weeks or more), and they take effect in only about 30% of patients. Therefore, in the field of severe depression treatment, there is an urgent medical need for faster-acting drugs (especially in suicidal patients) and drugs with new mechanisms of action, better therapeutic effect and less toxic side effects.

Chinese patents CN102850317A (with the application number 201210123842.7, hereinafter referred to as Patent A) and CN103687850A (with the application number 201280020049.2, hereinafter referred to as Patent B) disclose a substituted cinnamamide derivative, a preparation method, and use thereof in drugs for treating and preventing depressive psychiatric disorders, and disclose 13 specific compounds I-1 to I-13. In the patents, through experiments with "acquired despair" depression models in mice by tail suspension, experiments with depression models of blepharoptosis induced by reserpine, and experiments of forced swimming in mice, it is demonstrated that among the series of compounds, I-5, I-9, I-10, 1-11, 1-12 and 1-13, all can significantly shorten the immobility time of tail suspension in mice when administered at a dosage of 10 mg/kg for 7 days; compounds I-4, I-5, I-10, I-11, I-12 and I-13, when continuously administered at a dosage of 10 mg/kg for 7 days, have the effects of significantly antagonizing reserpine-induced body temperature decrease and akinesia in mice and improving the degree of eye closure, indicating that they all have certain regulation effect on 5-HT, NE and DA reuptake; and compounds I-5, I-10 and I-13 all can significantly shorten the immobility time of forced swimming in mice, and the effect of I-5 on the immobility time of forced swimming in mice is to a certain extent dosage-dependent. Among them, I-5, which has the best effect, is currently in the clinical research phase.

Chinese patent CN107011313A (with the application number 201710038281.3, hereinafter referred to as Patent C) discloses use of a substituted cinnamamide derivative in anti-anxiety. A total of 17 specific compounds are disclosed therein: II-3 (compound I-3 in Patents A and B), II-4 (compound I-4 in Patents A and B), II-5 (compound I-5 in Patents A and B), II-10 (compound I-10 in Patents A and B), II-11 (compound I-11 in Patents A and B), II-12 (compound I-12 in

Patents A and B), II-13, II-14, II-15, II-16, III-2, III-4, III-7, III-9, III-10, III-11, and III-13. In said patent, it is demonstrated by the elevated plus maze experiment on mice that the 17 compounds, when administered at a dosage of 10 mg/kg for 7 days, can increase, to varying degrees, the number of mice's entries into the open arm and prolong the time of stay of the mice in the open arm in the elevated plus maze test; and it is demonstrated by the rat drinking conflict experiment that the 17 compounds, when administered at a dosage of 5 mg/kg for 10 days, all can increase, to varying degrees, the number of times of drinking water of rats in the punishment period.

## Summary

In the present application, many compounds in the above patents are studied, and it is found that although most compounds have excellent therapeutic effect, they have low bioavailability and take effect slowly.

In a first aspect, the present application provides a faster-acting and more bioavailable antidepressant and anxiolytic substituted cinnamamide compound, or a solvate thereof, or a pharmaceutically acceptable salt thereof, the substituted cinnamamide compound having the following structural formula:

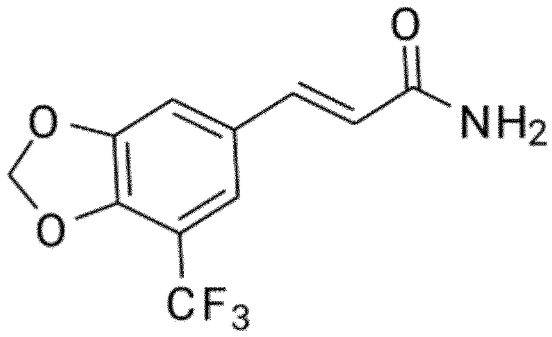

M2.

In a second aspect, the present application provides a pharmaceutical composition comprising the above-described substituted cinnamamide compound, or a solvent thereof, or a pharmaceutically acceptable salt thereof.

In a third aspect, the present application provides a preparation method of the above-described substituted cinnamamide compound.

In a fourth aspect, the present application provides use of the above-described substituted cinnamamide compound in the preparation of antidepressant, anxiolytic, or antidepressant and anxiolytic drugs.

In a fifth aspect, the present application provides use of the above-described compound in combination with one or more additional antidepressant or anxiolytic drugs in preparation of antidepressant, anxiolytic, or antidepressant and anxiolytic drugs.

In a sixth aspect, the present application provides a method for preventing or treating psychiatric disorders, the method including administering the above-described compound to a patient in need thereof.

## Brief Description of Drawings

FIG. 1 shows that compound M2 has antidepressant-like effect: 24 h after administration, compound M2 significantly reduces immobility time of mice, which exhibits an obvious dosage effect, and has significant effect when the dosage is 30 mg/kg.

FIG. 2 shows the effect of compound M2 on prefrontal excitatory synaptic transmission: it shows the effect of compound M2 at concentrations of 10 nM, 10 μM and 150 μM on sEPSC of pyramidal neurons in a prefrontal PrL brain area; the upper figures are schematic diagrams of sEPSC waveforms, and the lower figures are histograms after data statistics.

FIG. 3 shows that compound M2 enhances prefrontal excitatory synaptic transmission: at various concentrations, compound M2 increases sEPSC distribution frequency in prefrontal PrL brain area, in an obvious concentration-dependent manner; but does not have a significant effect on sEPSC distribution amplitude, wherein 150 μM com-

pound M2 can significantly enhance the sEPSC distribution frequency.

FIG. 4 shows that dopamine D2R receptor antagonist Sulpride blocks enhancement effect of compound M2 on prefrontal sEPSC: D2R antagonist sulpride can block D2R receptor at a working concentration of 10 μM, and the results show that blocking D2R can block the enhancement effect of 150 μM compound M2 on sEPSC distribution frequency.

FIG. 5 shows that dopamine D1R receptor antagonist SCH23390 blocks enhancement effect of compound M2 on prefrontal sEPSC: D1R receptor antagonist SCH23390 can block D1R receptor at a working concentration of 10 μM, and the results show that blocking D1R can block the enhancement effect of 150 μM compound M2 on sEPSC distribution frequency.

FIG. 6 shows that compound M2 activates mTOR-related signaling pathway: figure a shows a change in prefrontal related signaling protein 30 min after a single administration; and figure b shows a change in prefrontal related signaling protein 24 h after a single administration.

FIG. 7 shows inhibitory effect ($IC_{50}$) of compounds I-5 and M2 on DA, 5-HT, and NA uptake by rat brain synaptosomes.

FIG. 8 shows an average drug concentration-time curve after intragastric and intravenous administration of compound M2 in rats.

FIG. 9 shows an average drug concentration-time curve after intragastric and intravenous administration of compound I-5 in rats.

Detailed Description

In an embodiment of the first aspect, the present application provides a substituted cinnamamide compound, having a structural formula as follows:

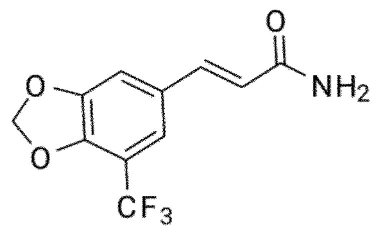

M2.

Further, the substituted cinnamamide compound of the present application may also be present in a form of a solvate.

Further, the substituted cinnamamide compound of the present application may also be present in a form of a pharmaceutically acceptable salt.

In an embodiment of the second aspect, the present application provides a pharmaceutical composition comprising the substituted cinnamamide compound of the present application, or a solvate thereof, or a pharmaceutically acceptable salt thereof.

The pharmaceutical composition of the present application may be in any doseable pharmaceutical form, e.g., tablets, sugar-coated tablets, film-coated tablets, enteric-coated tablets, capsules, hard capsules, soft capsules, oral liquids, oral lozenge, granules, pills, pulvises, pastes, pellets, suspensions, powders, solutions, injections, suppositories, soft ointments, hard ointments, creams, sprays, drops or patches.

The pharmaceutical composition of the present application is preferably in a form of a unit dosage pharmaceutical preparation.

When the pharmaceutical composition of the present application is made into a preparation, a unit dosage of the preparation may contain 0.1-1000 mg of the active pharmaceutical ingredient of the present application, and a balance of a pharmaceutically acceptable carrier. The pharmaceutically acceptable carrier may be 0.01-99.99% by weight of a total weight of the preparation.

When the pharmaceutical composition of the present application is administered, the mode of administration

and dosage are determined according to the conditions of the patient, e.g., 1-3 times a day, 1-10 tablets at a time, etc.

Preferably, the pharmaceutical composition of the present application is an oral preparation or an injection.

The oral preparation is selected from one of capsules, tablets, dripping pills, granules, concentrated pills, oral liquids, and mixtures.

The injection is selected from one of injection fluids, freeze-dried powder injections and liquid drug injections.

The orally administered preparation of the pharmaceutical composition of the present application may contain commonly used excipients, such as adhesives, fillers, diluents, tableting binders, lubricants, disintegrants, colorants, flavoring agents or wetting agents, and the tablets may be coated if necessary.

Suitable fillers include cellulose, mannitol, lactose or other similar fillers. Suitable disintegrants include starch, polyvinylpyrrolidone, or starch derivatives, preferably sodium starch glycolate. A suitable lubricant is magnesium stearate. A suitable wetting agent is sodium dodecyl sulfate.

The pharmaceutical composition of the present application may be prepared as a solid oral composition by commonly used methods such as mixing, filling, and tableting. Repeated mixing allows active substances to be distributed throughout the composition in which lots of fillers are used.

An oral liquid preparation may be in a form of an aqueous or oily suspension, solution, emulsion, syrup, or elixir, or a dried product that may be reconstituted in water or other suitable carriers before use. Such a liquid preparation may contain conventional additives such as suspending agents, e.g., sorbitol, syrup, methyl cellulose, gelatin, hydroxyethyl cellulose, carboxymethyl cellulose, aluminum stearate gel, or hydrogenated edible fat; emulsifiers, e.g., lecithin, sorbitan-oleate, or Arabic gum; non-aqueous carriers (which may include edible oils), e.g., almond oil, fractionated coconut oil, oily esters such as glycerol esters, propylene glycol, or ethanol; preservatives, e.g., p-hydroxybenzoate, propyl p-hydroxybenzoate or sorbic acid; and, if desired, conventional flavoring agents or colorants.

For injections, a prepared liquid unit dosage form contains active substance of the present application and a sterile carrier. The compound may be suspended or dissolved, depending on the carrier and concentration. A solution is usually prepared by dissolving active substance in a carrier, filtering and sterilizing it and then filling it in a suitable vial or ampoule, and then sealing it. Adjuvants such as a local anesthetic, a preservative and a buffer may also be dissolved in this carrier. In order to improve the stability, the composition may be frozen after being filled in vials, and water may be removed under vacuum.

When the pharmaceutical composition of the present application is prepared into a medicament, a suitable pharmaceutically acceptable carrier may be added selectively, and the pharmaceutically acceptable carrier is selected from one or more of: mannitol, sorbitol, sodium metabisulfite, sodium bisulfite, sodium thiosulfate, cysteine hydrochloride, mercaptoacetic acid, methionine, vitamin C, disodium EDTA, calcium sodium EDTA, carbonate, acetate, and phosphate of a monovalent alkali metal, or an aqueous solution thereof, hydrochloric acid, acetic acid, sulfuric acid, phosphoric acid, amino acid, sodium chloride, potassium chloride, sodium lactate, xylitol, maltose, glucose, fructose, dextran, glycine, starch, sucrose, lactose, mannitol, silicon derivatives, cellulose and derivatives thereof, alginate, gelatin, polyvinylpyrrolidone, glycerol, Tween 80, agar, calcium carbonate, calcium bicarbonate, surfactants, polyethylene glycol, cyclodextrin, β-cyclodextrin, phospholipid materials, kaolin, talc, calcium stearate, magnesium stearate, etc.

In addition to the substituted cinnamamide compound of the present application, the pharmaceutical composition of the present application may also include one or more of the following drugs for prevention and treatment of psychiatric disorders: e.g., nefazodone, sulpiride, alprazolam, lorazepam, buspirone, tandospirone, methylphenidate, fluoxetine, paroxetine, sertraline, citalopram, lexapro, fluvoxamine, reboxetine, venlafaxine, flupenthixol, melitracen, Neurostan, etc.

In an embodiment of the third aspect, the present application provides a preparation method of the substituted cinnamamide compound of the present application, the substituted cinnamamide compound being also referred to as compound M2, the preparation method being:

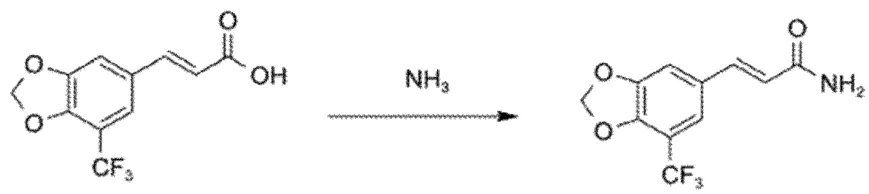


Intermediate A M2

reacting (E)-3-(3',4'-methylenedioxy-5'-trifluoromethyl-phenyl)-acrylic acid (i.e. intermediate A) with $NH_3$ to give compound M2.

In some embodiments of the third aspect, in the preparation method of the present application, $NH_3$ may be ammonia water ($NH_3$-$H_2O$).

In some embodiments of the third aspect, in the preparation method of the present application, the reaction is carried out in an organic solvent, said organic solvent includes dichloromethane (DCM).

In some embodiments of the third aspect, in the preparation method of the present application, (E)-3-(3',4'-methylenedioxy-5'-trifluoromethyl-phenyl)-acrylic acid (i.e. intermediate A) may be activated with an reagent of sulfonyl chlorination, such as oxalyl chloride or thionyl chloride, and then reacted with $NH_3$ to give compound M2.

In some embodiments of the third aspect, in the preparation method of the present application, the preparation may follow a reaction route as follows:

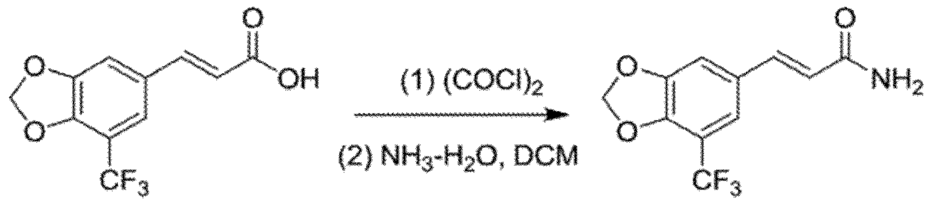


Intermediate A M2

wherein the reaction raw material is (E)-3-(3',4'-methylenedioxy-5'-trifluoromethyl-phenyl)-acrylic acid, and the raw material can be purchased from market, or can be prepared according to a method in a prior art literature.

The substituted cinnamamide compound of the present application may be prepared by the following method: dissolving (E)-3-(3',4'-methylenedioxy-5'-trifluoromethyl-phenyl)-acrylic acid (i.e. intermediate A) in dichloromethane, adding a catalytic amount of N,N-dimethylformamide, dropping oxalyl chloride under ice bath, stirring at room temperature until the completion of reaction of raw materials; concentrating and drying the reaction liquid, then adding dichloromethane to dissolve, dropping ammonia water under ice bath, reacting at room temperature until the completion of the reaction; and removing the solvent by evaporation under reduced pressure, then adding dilute hydrochloric acid to adjust it to be acidic, precipitating a solid, filtering and rinsing it with water to obtain a crude product, which is then subjected to silica gel column chromatography for purification to give compound M2.

In an embodiment of the fourth aspect, the present application provides use of compound M2 or a solvate thereof or a pharmaceutically acceptable salt thereof, or a pharmaceutical composition comprising compound M2 or a solvate thereof or a pharmaceutically acceptable salt thereof, in the preparation of antidepressant, anxiolytic, or antidepressant and anxiolytic drugs.

In some embodiments of the fourth aspect, the present application provides use of compound M2 or a solvate thereof or a pharmaceutically acceptable salt thereof, or a pharmaceutical composition comprising compound M2 or a solvate thereof or a pharmaceutically acceptable salt thereof in a drug.

In an embodiment of the fifth aspect, the present application provides use of compound M2 or a solvate thereof or a pharmaceutically acceptable salt thereof in combination with another antidepressant or anxiolytic drug, or use of compound M2 in combination with one or more additional antidepressant or anxiolytic drugs in the preparation of antidepressant, anxiolytic, or antidepressant and anxiolytic drugs; herein, the other antidepressant or anxiolytic drugs, or the additional antidepressant drugs or anxiolytic drugs may be selected from one or more of the following drugs for prevention and treatment of psychiatric disorders: e.g., nefazodone, sulpiride, alprazolam, lorazepam, buspirone, tandospirone, methylphenidate, fluoxetine, paroxetine, sertraline, citalopram, lexapro, fluvoxamine, reboxetine, venlafaxine, flupenthixol, melitracen, Neurostan, etc.

In an embodiment of the sixth aspect, the present application provides a method for preventing or treating psychiatric disorders, including administering compound M2 or a solvate thereof or a pharmaceutically acceptable salt thereof, or a pharmaceutical composition comprising compound M2 or a solvate thereof or a pharmaceutically acceptable salt thereof, to a patient in need thereof.

In some embodiments of the sixth aspect, the preventing or treating psychiatric disorders is anti-depression, anti-anxiety or anti-depression and anti-anxiety.

In some embodiments of the sixth aspect, the administration may be oral administration, injection or transdermal administration.

In some embodiments of the sixth aspect, the method for preventing or treating psychiatric disorders further includes combining with one or more additional antidepressant or anxiolytic drugs; here, the additional antidepressant drugs or anxiolytic drugs may be selected from one or more of the following drugs for prevention and treatment of psychiatric disorders: e.g., nefazodone, sulpiride, alprazolam, lorazepam, buspirone, tandospirone, methylphenidate, fluoxetine, paroxetine, sertraline, citalopram, lexapro, fluvoxamine, reboxetine, venlafaxine, flupenthixol, melitracen, Neurostan, etc.

## Specific examples

To further clarify the purpose, technical schemes and advantages of the present application, examples of the present invention will be described in detail below. It should be noted that the following examples of the present application and the features of the examples may be arbitrarily combined with each other provided that there is no conflict.

## Example 1

(E)-3-(3',4'-methylenedioxy-5'-trifluoromethyl-phenyl)-acrylic acid (15 g, 0.058 mol) was dissolved in 200 mL of dichloromethane, 0.1 mL of catalyst N,N-dimethylformamide was added, stirred in ice bath. Oxalyl chloride (18.3 g, 0.144 mol) was dissolved in 50 mL of dichloromethane, which was slowly dropped into the system, after the completion of dropping, the ice bath was removed, stirring was performed at room temperature until the completion of reaction of the raw materials; the reaction solution was concentrated and dried to give 16.5 g crude product; the crude product (10 g, 36 mmol) was added to 50 mL of dichloromethane and dissolved, ammonia water (12.6 g, 360 mmol) was slowly dropped under stirring in ice bath, and after the completion of dropping, the ice bath was removed, and reaction was carried out at room temperature until the completion of the reaction.

Concentrated hydrochloric acid was added to the system, pH was adjusted to be acidic, and dichloromethane was spun off under reduced pressure, followed by suction filtration until it was dried to give 8.67 g white crude product, which was recrystallized by ethanol-water (with a volume ratio of 1:2) to give 6.4 g compound M2.

## Experimental example 1: Study on the efficacy of compound M2 and functions on the brain area

The study objective of this experiment is to demonstrate the antidepressant effect of compound M2 and the brain area function mechanism through the following two experiments:

**1) Forced swimming experiment: also known as despair experiment, an experiment usually used to test depression-like behavior of mice**

Animal: C57 BL/6J male mice (18-20 g)

| Group | Dosage (mg/kg) | Administration volume (ml/kg) | Number of animals | Mode of administration | Forced swimming test time |
|---|---|---|---|---|---|
| Solvent control (Vel) | - | 10 | 10 | i.p | 24 h after a single administration |
| Esketamine (Esketa) | 10 | 10 | 10 | i.p | |
| Fluoxetine (Flx) | 10 | 10 | 10 | i.p | |
| Low dosage M2 | 3 | 10 | 10 | i.p | |
| Medium dosage M2 | 10 | 10 | 10 | i.p | |
| High dosage M2 | 30 | 10 | 10 | i.p | |

Intraperitoneal injection (i.p)

**2) Brain slice electrophysiological experiment: for detecting effect of compound M2 on mPFC excitatory synaptic transmission**

- Animal: neonatal mice C57 BL/6J (20-30 days).
- Instrument: vibratome (*Leica*), brain slice electrophysiological system (*Axon*).
- Brain slice electrophysiological experiment method
- sEPSC experiment method: Neonatal C57 mice were anesthetized and then perfused with artificial cerebrospinal fluid (ACSF). After decapitation, brain tissue was taken quickly and sliced by vibratome, with a thickness of a brain slice being 300 μm. After the brain slices were incubated at 28°C for 1 h, whole-cell recordings of mPFC pyramidal neurons were made using a voltage clamp (-70 mV) through a brain slice electrophysiological system. The baseline recording time was 6 min, and then a drug to be tested was added through a perfusion system (the drug was dissolved using ACSF), and the recording time after adding the drug was 6-10 min. Recorded data was analyzed using Minianalysis software, and effects of the drug to be tested on sEPSC frequency and amplitude of medial prefrontal pyramidal neurons in C57 mice were compared.
- **Data statistics**

All data analysis was done using spss data processing software, and data was expressed by Mean ± s.e.m. One-way ANOVA was used to analyze the effect of compound M2 and the effect of compound M2 under the action of dopamine receptor antagonists on sEPSC current; and paired t test was used to analyze sEPSC experimental results. * was marked when $p<0.05$.

**3) Western blot: for detecting effect of compound M2 on a change in mPFC protein content**

• Animals: C57 BL/6J male mice (18-20 g), divided into four groups, i.e. the control group, fluoxetine group, compound M2 (30 mg/kg) group, and esketamine group.
• Primary antibodies for experimental use

| Primary antibody | Brand | Item number |
|---|---|---|
| p-mTOR | Cst | 2971s |
| mTOR | Cst | 2983s |
| p-TrkB | Cst | 4621s |
| TrkB | Cst | 4603s |
| PSD95 | Cst | 3450s |
| Synapsin 1 | Cst | 5297s |
| β-tubulin | Cst | 2128s |

**•Western blot experimental method:**

0.5h/24h after administration of C57 mice, brain tissue was sampled from the prefrontal brain area. After tissue samples were lysed and homogenized, quantification of proteins was performed using the BCA method, and changes in protein content in the prefrontal brain area 0.5 h/24 h after intraperitoneal administration of compound M2 were detected by steps of SDS-PAGE gel preparation, sample loading and electrophoresis, membrane transfer, sealing, primary antibody incubation, secondary antibody incubation, protein detection (development and fixation), etc.

**3) Experimental results**

**1. Compound M2 has antidepressant-like effect**

24 h after administration, compound M2 significantly reduced the immobility time of mice, which exhibited an obvious dosage effect, and had significant effect when the dosage was 30 mg/kg, as shown in FIG. 1. Compound M2 had a faster-acting antidepressant effect: two positive compounds were used as controls in the experiment, in which fluoxetine, a first-line clinical drug, took effect slowly, while esketamine took effect rapidly. The results of this experiment

showed that 24 h after a single administration, fluoxetine had no antidepressant-like effect, while compound M2 and esketamine both had antidepressant-like effect, suggesting that compound M2 had the potential of fast-acting antidepressant effect.

**2. Compound M2 significantly enhanced excitatory synaptic transmission in the medial prefrontal cortex (mPFC)**

During the experiment, 10 μM Bicuculine was added to the solution to block GABA receptor, so as to record sEPSC of pyramidal neurons. The experiment investigated effects of different concentrations of compound M2 on spontaneous excitatory postsynaptic potential (sEPSC) of pyramidal neurons in prefrontal PrL brain area, as shown in FIG. 2. The results showed that M2 increased sEPSC distribution frequency of prefrontal PrL brain area, in an obvious concentration-dependent manner; but did not have significant effect on sEPSC distribution amplitude, as shown in FIG. 3.

The increase in sEPSC distribution frequency suggested that compound M2 can enhance the release of excitatory glutamate neurotransmitters from prefrontal synapses. According to the targeting characteristics of compound M2, this increase may be caused by compound M2 enhancing synaptic transmission projected from midbrain to prefrontal monoamine neurotransmitters, or directly acting on prefrontal monoamine receptors. Prefrontal excitatory synaptic transmission is closely related to antidepressant effect, and novel antidepressant drugs such as ketamine can all enhance prefrontal excitatory synaptic transmission.

**3. Compound M2 increased prefrontal excitatory synaptic transmission through dopamine D2R receptor**

1) Dopamine D2R receptor blocked enhancement effect of compound M2 on prefrontal sEPSC

This experiment investigated whether prefrontal dopamine receptor affects the effect of compound M2 on prefrontal cortex. Sulpride, an antagonist of dopamine receptor D2R, was used to block D2R receptor in the experiment. The results showed that blocking dopamine D2R receptor can significantly block the enhancement effect of compound M2 on sEPSC distribution frequency, suggesting that prefrontal D2R receptor may mediate excitatory synaptic transmission induced by M2 (as shown in FIG. 4).

2) Dopamine D1R receptor did not affect enhancement effect of compound M2 on prefrontal excitatory synaptic transmission

Dopamine D1R receptor antagonist SCH23390 was used. The results showed that blocking dopamine D1R receptor cannot block the enhancement effect of compound M2 on sEPSC distribution frequency, suggesting that prefrontal D1R receptor may not participate in excitatory synaptic transmission induced by compound M2 (as shown in FIG. 5).

**4. Compound M2 rapidly activated prefrontal synaptogenesis-related signaling pathway**

This experiment investigated the possible intracellular signaling pathway through which compound M2 exerts its antidepressant effect. Since activation of mTOR signaling pathway plays an important role in fast-acting antidepressant effect, this study investigated changes of upstream and downstream related proteins of mTOR signaling pathway. The results showed that medial prefrontal p-mTOR and p-TrkB contents can be increased 0.5 h after administration of compound M2 (FIG. 6.a), with the total content remaining unchanged. Literature suggested that the mTOR signaling pathway is related to the mechanism of rapid depressive disease, and the fast-acting antidepressant effect of ketamine may be related to increasing phosphorylated mTOR content and activating TrkB protein (BDNF receptor). These results suggested that the antidepressant effect of compound M2 may also be related to the activation of mTOR and TrkB protein.

The activation of the mTOR signaling pathway further enhances the increase in the expression level of synapse-related proteins. FIG. 6.b shows that compound M2 significantly increased the content of medial prefrontal synaptogenesis-related protein PSD95 24h after single administration. This result was consistent with the finding in the electrophysiological experiment that compound M2 enhanced prefrontal synaptic transmission function. PSD95 is a postsynaptic density protein, which is related to neuroplasticity. Literature studies (Shinohara R, Aghajanian G K, Abdallah C G. Neurobiology of the Rapid Acting Antidepressant Effects of Ketamine: Impact and Opportunities[J]. Biological Psychiatry, 2020.) showed that the exertion of antidepressant and anxiolytic effect of ketamine was related to increase in the expression level of PSD95 protein.

Studies have shown (Pizzagalli D A, Roberts A C. Correction: Prefrontal cortex and depression[J]. Neuropsychopharmacology, 2021:1-1.) that clinically, depressed patients may have morphological features of prefrontal atrophy, and in basic experimental studies, modeled mice under chronic social stress exhibit reduced spine density of neurons in the fifth layer of prefrontal cortex, reduced EPSC frequency of neuronal excitatory postsynaptic current, and reduced EPSC frequency, reducing synaptic transmission of prefrontal excitatory neurotransmitters. Prefrontal excitatory synaptic

transmission is closely related to antidepressant and anxiolytic effects, and ketamine, fluoxetine, etc. all can enhance prefrontal excitatory synaptic transmission. Therefore, this experiment focused on the antidepressant and anxiolytic effect of compound M2, and studied the effect of the compound on glutamatergic system of excitatory neurotransmitter in brain, in conjunction with the pharmacological characteristics of compound M2. Using the experiment of prefrontal brain slices which more resemble the physiological conditions, it was found that compound M2 could promote the release of glutamate neurotransmitters and enhance the function of glutamate receptors, suggesting that compound M2 enhanced prefrontal excitability, in a concentration-dependent manner.

Based on the above results, it is indicated that compound M2 exerted its fast-acting antidepressant pharmacological effect mainly by acting on the glutamatergic neurotransmitter system and enhancing excitatory glutamatergic synaptic transmission to improve prefrontal excitability. In addition, compound M2 rapidly activated prefrontal mTOR signaling pathway. The results suggested that compound M2 had a fast-acting antidepressant effect and a novel antidepressant mechanism of enhancing synaptic transmission and brain excitability, showing better antidepressant potential than clinical monoamine drugs.

**Experimental Example 2 Inhibitory effect of compound 1-5 and compound M2 on monoamine reuptake of synaptosomes in rat brains**

At present, it is believed that absolute or relative deficiencies of the central monoamine neurotransmitters 5-hydroxytryptamine (5-HT), norepinephrine (NA) and dopamine (DA) are closely related to depression and anxiety. This experiment was designed to evaluate, by the method of *in vitro* monoamine reuptake, the inhibitory effect of two samples of tested compound I-5 and compound M2 on reuptake of 5-HT, NA and DA in rat brain synaptosomes. The preparation of *in vitro* SD rat brain synaptosomes and isotopic tracer method were employed to investigate $IC_{50}$ of inhibition of monoamine reuptake of DA, 5-HT and NA in two tested samples, in order to explore the possible mechanism of antidepressant and anxiolytic effects of compound I-5 and compound M2.

**I. Experimental materials**

**1. Experimental animals:** SD rats, male, weight: 200-220 g

**2. Experimental samples:**

As can be known according to the recordation in the experimental part in the description of Patents A and B, among all the compounds, compound I-5 had good results in all experiments, so compound I-5 was selected as control to compare the inhibitory effect of compound M2 on monoamine reuptake in rat brain synaptosomes.

**Test product 1**

| | |
|---|---|
| Name: | Compound I-5 |
| Batch number: | PS01068-6-C-S |

The structure was as follows:

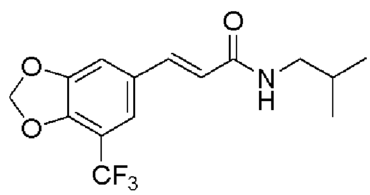

**I-5**

**Test product 2**

Name: Compound M2
Batch number: 20171208

**Positive control product 1**

Name: 6-Hydroxydopamine hydrobromide
Batch number: 0000051379
Positive control: 100 μM can inhibit 100% $^3$H-DA reuptake by synaptosomes

**Positive control product 2**

Name: Fluoxetine hydrochloride
Batch number: WXBC7489V
Positive control: 100 μM can inhibit 100% $^3$H-5-HT reuptake by synaptosomes

**Positive control product 3**

Name: Desipramine hydrochloride
Batch number: MKCH5352
Positive control: 100 μM can inhibit 100% $^3$H-NA reuptake by synaptosomes

**3 Preparation of test products and positive control products, etc.**

**3.1 Preparation of test products**

Test products were weighed (a weighing error of ±1% was allowable) and dissolved in DMSO to prepare 10 mM stock solution, which was stored at -20°C.

| Tested sample | Concentration (mM) | Amount weighed (mg) | Solvent added (mL) |
|---|---|---|---|
| **Compound I-5** | **10** | **5.63** | **1.79*** |
| **Compound M2** | **10** | **4.48** | **1.73*** |

Before the experiment, by the doubling dilution method, it was diluted with Kreb's solution to 100-fold working solution required for the detection concentration (1 nM-100 μM).

Conversion formula:

$$\text{detection concentration} = \frac{\text{volume of test compound in reaction tube X concentration of working solution}}{\text{final volume of the reaction tube}}$$

The preparation was as follows:

Detection concentration (100 μM)-stock solution 10 mM:10 μl*

Detection concentration (10 μM)-working solution concentration 1 mM:100 μl*Kreb's → final volume to 1000 μl①

Detection concentration (1 nM)-working solution concentration 100 μM:100 μl①Kreb's → final volume to 1000 μl②

Detection concentration (100 nM)-working solution concentration 10 μM: 100 μl②Kreb's→ final volume to 1000 μl③

Detection concentration (10 nM)-working solution concentration 1 nM:100 μl③Kreb's → final volume to 1000 ul④

Detection concentration (1 nM)-working solution concentration 100 nM:100 μl④Kreb's → final volume to 1000 μl⑤

Note: vortex mixing and light avoidance were required in each step of preparation. The working solution used in each experiment was prepared *in situ* before the experiment.

**3.2 Preparation of positive control products**

Control products were weighed (a weighing error of ±1% was allowable) and dissolved in double distilled water to prepare 10 mM stock solution which was stored at -20°C.

| Positive control product | Monoamine receptor control | Concentration (mM) | Amount weighed (mg) | Solvent added (mL) |
|---|---|---|---|---|
| 6-hydroxydopamine | DA | 10 | 2.25 | 0.899 |
| Fluoxetine | 5-HT | 10 | 3.66 | 1.06 |
| Desipramine | NA | 10 | 3.25 | 1.07 |

**II. Experimental method**

**1. Preparation of brain synaptosomes**

Synaptosomes were separated using methods in literature to prepare brain synaptosomes. SD rats were rapidly decapitated with a small animal decapitator. After decapitation, brains were taken out quickly and precooled in an ice-water mixture, and the pia mater and vascular tissue were removed. Brain tissue was taken out. 10 times volume (ml/g) of 10 mM Tris HCl buffer (containing 0.32 M sucrose, pH 7.4) was added, an ultrasonic cell pulverizer was used for homogenization (maintaining at a low temperature), and the brain tissue was homogenized and centrifuged for 10 min (4°C, 1,000 g). After balanced centrifugation (1500 g, 10 min) at 4°C, precipitate was discarded, then supernatant was taken, and centrifugation (20000 g) was performed again for 30min. The supernatant was discarded and the precipitate, i.e. crude extract of synaptic corpuscles, was retained. The precipitate was further suspended in 0.32 M cold sucrose solution and then carefully laid on 1.2 M and 0.8 M (10ml each) cold sucrose gradient solutions which had been laid sequentially on the bottom of the tube, followed by balanced centrifugation (38000g) at 4°C for 60 min. A suspension band at the 0.8-1.2 M sucrose interface was carefully collected with a puncture needle, which was placed in 10 ml of 0.32 M cold sucrose solution and mixed well, and equilibrium centrifuged (20000 g) at 4°C for 30 min. The precipitate was refined brain synaptosomes. The precipitate was suspended in a small amount of Kreb's buffer (NaCl 118 mM, KCl 4.7 mM, $CaCl_2$ 2.5 mM, $MgSO_4$ 1.2 mM, $KH_2PO_4$ 1.2 mM, NaHCOs 25 mM and Glucose 11.1 mM, pH 7.2-7.4), BCA method was used for protein quantification, and the operation steps were as described in the description.

**2. Reuptake of monoamine**

Referring to relevant methods in literature, with optimization by the inventor in laboratory, the experimental method was briefly described as follows: adding 950 μl of precooled Kreb's buffer into a reaction tube, then adding 30 μl of synaptic corpuscle suspension, then adding 10 μl of the compound to be tested (operated on ice), vortex mixing well, followed by water bath at 37°C for 5min; taking out the reaction tube and placing it on ice, then adding 10 μl substrate ($^3$H-DA or $^3$H-5HT or $^3$H-NA; final reaction concentration: 10nM), vortex mixing well, followed by water bath at 37°C for 5 min; then taking out the reaction tube and placing it on ice quickly, adding 3 ml of precooled Kreb's buffer to stop the reaction, collecting with Millipore cell sample collector, followed by rapid suction filtration through GF/C glass fiber filter membrane and elution by 3 ml eluent (50 mM Tris-HCl, PH 7.4) for 3 times, removing the filter membrane, drying with a microwave oven for 5-6 min, placing the filter membrane into 1.5 ml centrifuge tube, and adding 500 μl of fat-soluble scintillation solution; and standing away from light for 30 min or more, and measuring radioactivity intensity (cpm value) by counting.

The percentage inhibition of isotope ligand binding by each compound was calculated according to the following

equation:

Inhibition rate (I%) = (total binding tube cpm-compound cpm)/(total binding tube cpm-nonspecific binding tube cpm) × 100%

Three duplicate tubes of compound were used in each experiment, with the experiment repeated three times separately.

**III. Experimental results**

**1. Calculation of $IC_{50}$**

1) The concentration of the compound was detected to be X and the inhibition rate was Y.
2) Logarithm of X was taken, X'= Log(X) to form new data.
3) A nonlinear curve fitting was done to the new data, and after fitting, it was substituted into the following equation to calculate the value of $IC_{50}$. The above steps were fitted by software GraphpadPrism.
4)

Equation

Equation: Sigmoidal dose-response ( variable slope )

Y=Bottom+ ( Top-Bottom ) /(1+10^((LogIC50-X)*HillSlope))

: X is the logarithm of concentration. Y is the response

: Y starts at Bottom and goes to Top with a sigmoid shape

: This is identical to the "four parameter logistic equation"

| Compound | DA inhibition rate% $IC_{50}$ (nM) | 5-HT inhibition rate% $IC_{50}$ (nM) | NA inhibition rate% $IC_{50}$ (nM) |
|---|---|---|---|
| I-5 | 26.02±1.33 | 18.52±2.47 | 17.21±1.59 |
| M2 | 37.28±1.97 | 98.87±2.42 | 23.54±3.56 |

**2. Experiment summary**

Monoamine reuptake inhibition is one of the main targets of antidepressant and anxiolytic drugs. The test compound I-5 and compound M2 had different degrees of inhibitory effects on DA, 5-HT, and NA reuptake of rat brain synaptosomes, and the inhibitory effect of compound I-5 was equivalent to that of compound M2, suggesting that compound I-5 and compound M2 exerted antidepressant and anxiolytic effects by inhibiting DA, 5-HT, and NA reuptake.

The values of $IC_{50}$ of compound I-5 for DA, 5-HT, and NA reuptake in rat brain synaptosomes were: 26.02±1.33 nM, 18.52±2.47 nM and 17.21±1.59 nM, respectively.

The values of $IC_{50}$ of compound M2 for DA, 5-HT, and NA reuptake in rat brain synaptosomes were: 37.28±1.97 nM, 98.87±2.42 nM and 23.54±3.56 nM, respectively.

**Experimental Example 3: Study on antidepressant effect of compound 1-5 and compound M2**

"Behavioral despair" models were developed by Porsolt et al. in 1977, including rats and mice forced swimming models and mice tail suspension models, which were acute stress models. Mice tail suspension experiment was a simple

and easy-to-implement experimental method for evaluating antidepressant drugs introduced by Stern et al. in 1985, and its principle was the same as that of forced swimming "immobility" experiment. The upside-down mice struggled to overcome the abnormal body position, but after a certain period of activity, the animals showed intermittent immobility due to "disappointment".

In this experiment, whether the to-be-tested compound I-5 and compound M2 affected tail suspension behavior of mice in the "behavioral despair" experiment and whether they had the effect of shortening the immobility time of mice were observed, and the fast-acting effect of compound I-5 and compound M2 was comparatively studied.

**I. Experimental materials**

**1. Tested sample 1**

Name or code: Compound I-5

Test product No.: YLS-2021-CMI1203-002

Batch No.: C16101006-C17001M

Preparation method: the solvents were Tween 80 + water, the compound was fully emulsified with Tween 80 in a volume of 1.5% of the final volume, and water was added to the final volume, and the temporary storage conditions after preparation were as follows: 4°C refrigerator.

**2. Tested sample 2**

Name or code: Compound M2

Test product No.: YLS-2021-CMI 1203-01-001

Batch No.: 20201029

Preparation method: the solvents were Tween 80 + water, the compound was fully emulsified with Tween 80 in a volume of 1.5% of the final volume, and water was added to the final volume, and the temporary storage conditions after preparation were as follows: 4°C refrigerator.

**II. Experimental animals**

C57 BL/6 mice, 40, SPF grade, male, 4-5 weeks.

**III. Experimental method**

**1. Animal grouping and dosage design basis**

40 mice were divided into 4 groups according to the Excel complete random grouping method, with 10 animals in each group. Pre-experiment results suggested that compound M2 was able to reduce the immobility time of mice in the forced swimming experiment in a dosage-dependent manner 24 h after administration, and the effect was obvious at the dosage of 30 mg/kg. Therefore, in this experimental study, with 30 mg/kg as the low dosage of compound M2, 60 mg/kg was set as the high dosage of compound M2 to conduct research in comparison with 60 mg/kg compound I-5. Details of grouping and administration were shown in Table 1.

**Table 1 Animal grouping and administration**

| Group | Dosage | Number of animals | Mode of administration |
|---|---|---|---|
| Blank control group (normal animals) | Purified water + Tween 80 | 10 | Oral intragastric administration (i.g.) |
| Compound 1-5 group | 60 mg/kg | 10 | |
| Compound M2 high dosage group | 60 mg/kg | 10 | |
| Compound M2 low dosage group | 30 mg/kg | 10 | |

**2. Route and time of administration**

Each test group was administered intragastrically (i.g.) at 0.2 ml/10 g body weight.

**3. Index detection**

Mice tail suspension test was carried out 30 min after a single oral administration, and the tail suspension lasted for 6 min. The immobility time of mice within 6 min was recorded.

**4. Statistical method**

Data was expressed as mean±standard (mean±SD) difference, and statistics of data differences were made by one-way analysis of variance (ANOVA) or nonparametric test, and differences between groups were determined by $P<0.05$.

**IV. Experimental results**

The results of single administration test in mice showed that the immobility time of mice in tail suspension could be significantly shortened 30 min after a single administration of 60 mg/kg compound M2, $p<0.05$ compared with the blank group; although there was a certain tendency to reduce the immobility time of mice in tail suspension 30 min after a single administration of 30 mg/kg compound M2, no statistically significant difference was observed; and the immobility time of mice in tail suspension was not significantly affected 30 min after a single administration of 60 mg/kg compound I-5 (see Table 2 for details). The above results suggested that compound M2 had a fast-acting antidepressant effect, and compound M2 had significant antidepressant effect which took effect rapidly, and was significantly superior to compound I-5 in terms of fast-acting effect.

**Table 2. Effect of compound 1-5 and compound M2 at a single administration on tail suspension experiment in C57 mice (n=10, $\bar{x} \pm s$)**

| Group | Drug dosage | Immobility time within 6 min (s) |
|---|---|---|
| Blank control group | Purified water+Tween 80 | 199.3±29.2 |
| Compound 1-5 group | 60 mg/kg | 218.0±35.3 |
| Compound M2 high dosage group | 60 mg/kg | 171.8+21.7**### |
| Compound M2 low dosage group | 30 mg/kg | 183.9±43.5 |
| Note: Compared with the blank control group, **P<0.05; and compared with compound I-5 group, ###P<0.01 | | |

**Experimental example 4: Study of absolute bioavailability of compound 1-5 and compound M2 in rats**

**1. Purpose of the experiment**

A LC-MS/MS method for determination of compound I-5 and compound M2 in rat plasma was established through experiments to investigate the pharmacokinetic characteristics and absolute bioavailability of compounds in rats.

## 2. Experimental materials

### 2.1 Experimental animals

20 SPF-grade SD male rats (body weight 220±20 g), purchased from Beijing Vital River Laboratory Animal Technology Co., Ltd.

### 2.2 Test products

Compound I-5 bulk drug, batch No.: C16101006-C(20170814)M, content: 99.6%, provided by Tasly Pharmaceutical Group Co., Ltd.
Compound M2 bulk drug, batch No.: 20201029, content: 99.8%, provided by Tasly Pharmaceutical Group Co., Ltd.

## 3. Experimental method

### 3.1 Preparation and dosage of test products

| PK experiment | | | | | | |
|---|---|---|---|---|---|---|
| Group | | Daily dosage (mg/kg/d) | intragastric volume (mL/kg/ dose) | Number of animals | Preparation method |
| A | Compound M2 single intragastric administration group | 12.5 | 10 | 5 | Preparation method: measuring 25 mg test product precisely, then adding 1.5% Tween and 20 mL of double purified water, and mixing well |
| B | Compound M2 single intravenous administration group | 2.5 | 2 | 5 | Preparation method: measuring 5 mg test product precisely, then adding 4mL of double purified water (containing 10% ethanol), and mixing well |
| C | Compound 1-5 single intragastric administration group | 12.5 | 10 | 5 | Preparation method: measuring 25 mg test product precisely, then adding 1.5% Tween and 20 mL of double purified water, and mixing well |
| D | Compound 1-5 single intravenous administration group | 2.5 | 2 | 5 | Preparation method: measuring 5 mg test product precisely, then adding 4mL of double purified water (containing 10% ethanol), and mixing well |

### 3.2 Determination method

#### 3.2.1 Liquid mass condition of compound M2 (positive spectrum)

3.2.1.1 Liquid phase conditions

Chromatographic column: waters BEH Cis 1.7 μm; 2.1×100 mm Column

Internal standard: D-I-5 (deuterated compound I-5, with the following structural formula)

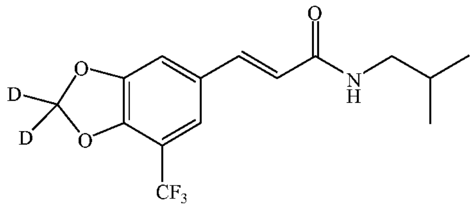

D-I-5

Mobile phase: 0.1% formic acid aqueous solution (A), acetonitrile (B); isocratic elution (A/B:58:42 v/v)

Flow rate: 0.4 ml/min; column temperature: 40°C; injection volume: 4 μL

3.2.1.2 Mass spectrometry conditions

Mode of ion detection: selected reaction monitoring (MRM)

Mode of ionization: electrospray ionization (ESI)

Ion polarity: positive ions

Mass spectrometry parameters

| Target compound | ESI mode | Q1 | Q3 | CE |
|---|---|---|---|---|
| Compound 1-5 | ESI+ | 316.00 | 243.00 | -15V |
| Compound M2 | ESI+ | 260.00 | 243.10 | -15V |
| D-I-5 | ESI+ | 318.00 | 157.05 | -42V |

**3.3 Pharmacokinetic experiment**

20 SD rats were randomly divided into 4 groups, with 5 rats in each group. The rats were fasted for 10 h before administration and drank freely. 0.3 mL blood was taken from orbital vein before administration (0 h) and 0.083, 0.25, 0.5, 1, 2, 4, 6, 8, 10, and 24 h after administration, the blood was placed in an EDTA-K2 anticoagulant tube, which was shaken gently to mix well with the anticoagulant, placed on wet ice and centrifuged within 0.5 h.

Centrifugation: after 8000 rpm centrifugation for 5min, plasma was separated.

Storage: freeze storage at -20°C.

**3.4 Method for processing plasma samples**

50 μL plasma was taken, 50 μL D-I-5 (20 ng/mL dissolved in acetonitrile) was added, the mixture was whirled for 30 s, then 100 μL acetonitrile was added; the mixture was fully whirled for 2 min, and was centrifuged at 4°C at 12000 r/min for 3 min, 50 μL supernatant was taken, 50 uL aqueous solution was added for dilution, followed by whirling for 1min, and 4 μL sample was injected.

**4. Analysis of pharmacokinetic results**

The mean plasma concentration-time curves of rats after oral intragastric administration and intravenous administration were respectively shown in FIGS. 8 and 9. Data were processed using DAS 3.0 pharmacokinetic software (Chinese Professional Committee of Mathematical Pharmacology, Shanghai, China), and statistical moment parameters of the non-compartmental model were calculated, $C_{max}$ and $T_{max}$ being measured values. The plasma concentration data and pharmacokinetic parameters were shown in the table below.

**Table 3 Plasma concentration (ng/mL) in rats after a single intragastric administration of compound M2 (12.5 mg/kg)**

| Time (h) | Rat1 | Rat2 | Rat3 | Rat4 | Rat5 | Mean | Sd |
|---|---|---|---|---|---|---|---|
| 0 | BLOQ | BLOQ | BLOQ | BLOQ | BLOQ | / | / |
| 0.08 | 78.15 | 127.05 | 78.40 | 240.77 | 433.00 | 191.47 | 150.45 |
| 0.25 | 213.75 | 487.46 | 212.80 | 494.43 | 582.67 | 398.22 | 172.95 |
| 0.50 | 270.87 | 411.67 | 291.90 | 416.38 | 542.56 | 386.67 | 109.77 |
| 1 | 144.95 | 184.82 | 165.92 | 270.26 | 288.12 | 210.81 | 64.30 |
| 2 | 90.66 | 71.40 | 131.47 | 231.27 | 133.28 | 131.62 | 61.71 |
| 3 | 175.71 | 60.26 | 194.74 | 114.91 | 122.55 | 133.63 | 53.29 |
| 4 | 268.66 | 107.99 | 188.58 | 66.64 | 188.12 | 164.00 | 78.66 |
| 6 | 57.55 | 48.74 | 11.84 | 15.99 | 299.46 | 86.72 | 120.58 |
| 8 | BLOQ | 5.38 | BLOQ | BLOQ | 3.64 | 2.07 | 2.38 |

BLOQ: Below the Limit Of Quantification

**Table 4 Plasma concentration (ng/mL) in rats after a single intravenous injection of compound M2 (2.5 mg/kg)**

| Time (h) | Rat1 | Rat2 | Rat3 | Rat4 | Rat5 | Mean | Sd |
|---|---|---|---|---|---|---|---|
| 0 | BLOQ | BLOQ | BLOQ | BLOQ | BLOQ | / | / |
| 0.08 | 1088.43 | 1221.94 | 1589.64 | 991.98 | 1170.61 | 1212.52 | 228.08 |
| 0.25 | 574.71 | 742.62 | 807.81 | 497.57 | 574.42 | 639.42 | 129.93 |
| 0.50 | 281.81 | 435.1 | 406.94 | 201.98 | 248.89 | 314.96 | 101.40 |
| 1 | 74.30 | 130.96 | 92.59 | 39.74 | 57.32 | 78.98 | 35.06 |
| 2 | 4.62 | 12.27 | 2.23 | BLOQ | 2.72 | 5.46 | 4.65 |
| 3 | 2.035 | 4.403 | BLOQ | BLOQ | BLOQ | 3.21 | 1.67 |
| 4 | BLOQ | BLOQ | BLOQ | BLOQ | BLOQ | / | / |
| 6 | BLOQ | BLOQ | BLOQ | BLOQ | BLOQ | / | / |
| 8 | BLOQ | BLOQ | BLOQ | BLOQ | BLOQ | / | / |

**Table 5 Plasma concentration (ng/mL) in rats after a single intragastric administration of 12.5 mg/kg compund I-5**

| Time (h) | Rat1 | Rat2 | Rat3 | Rat4 | Rat5 | Mean | Sd |
|---|---|---|---|---|---|---|---|
| 0 | BQL | BQL | BQL | BQL | BQL | / | / |
| 0.08 | 140 | 3.59 | 70.1 | 214 | 164 | 118.3 | 82.5 |
| 0.25 | 706 | 7.11 | 371 | 389 | 361 | 366.8 | 247.5 |
| 0.50 | 735 | 4.71 | 416 | 268 | 246 | 333.9 | 268.4 |
| 1 | 450 | BQL | 391 | 92.3 | 132 | 266.3 | 180.4 |
| 2 | 224 | BQL | 134 | 35.8 | 43.0 | 109.2 | 88.6 |
| 3 | 84.5 | BQL | 58.4 | 11.3 | 23.6 | 44.4 | 33.3 |
| 4 | 27.3 | BQL | 17.3 | 10.7 | 6.16 | 15.3 | 9.2 |
| 6 | 5.64 | BQL | 3.20 | 5.66 | 12.2 | 6.6 | 3.9 |
| 8 | BQL | BQL | BQL | BQL | BQL | / | / |

BLOQ: Below the Limit Of Quantification

**Table 6 Plasma concentration (ng/mL) in rats after a single intravenous injection of 2.5 mg/kg compound 1-5**

| Time (h) | Rat1 | Rat2 | Rat3 | Rat4 | Rat5 | Mean | Sd |
|---|---|---|---|---|---|---|---|
| 0 | BQL | BQL | BQL | BQL | BQL | / | / |
| 0.08 | 1750 | 1150 | 659 | 865 | 1350 | 1154.8 | 424.9 |
| 0.25 | 628 | 247 | 540 | 260 | 291 | 393.2 | 177.7 |
| 0.50 | 243 | 75.7 | 225 | 95.9 | 120 | 151.9 | 76.8 |
| 1 | 103 | 24.9 | 80.2 | 52.0 | 41.6 | 60.3 | 31.2 |
| 2 | 25.1 | 6.66 | 19.3 | 15.2 | 17.7 | 16.8 | 6.7 |
| 3 | 8.95 | 2.49 | 9.76 | 5.45 | 7.89 | 6.9 | 3.0 |
| 4 | 3.01 | BQL | 3.38 | 2.60 | 3.08 | 3.0 | 0.3 |
| 6 | BQL | BQL | BQL | BQL | BQL | | |
| 8 | BQL | BQL | BQL | BQL | BQL | | |

**Table 7. Pharmacokinetic parameters (Mean±SD) (n=5) in rats after intragastric administration and intravenous administration of compound M2**

| Parameter | Unit | intravenous administration (2.5 mg/kg) | intragastric administration (12.5 mg/kg) |
|---|---|---|---|
| | | M2 | M2 |
| $t_{1/2}$ | h | 0.24±0.03 | 1.03±0.34 |
| $C_0/C_{max}$ | ng/mL | 1641.93±319.23 | 425.47±136.97 |
| $t_{max}$ | h | - | 0.35±0.14 |
| $AUC_{0-t}$ | ng.h/mL | 529.41±129.86 | 1080.11±373.43 |
| $AUC_{0-\infty}$ | ng.h/mL | 532.41±126.82 | 1122.48±366.77 |
| $MRT_{0-\infty}$ | h | 0.31±0.06 | 2.52±0.61 |
| F (bioavailabiliy, %) | | - | 40.80 |

**Table 8. Pharmacokinetic parameters (Mean±SD) (n=5) in rats after intragastric administration and intravenous administration of compound I-5**

| Parameter | Unit | intravenous administration (2.5 mg/kg) | intragastric administration (12.5 mg/kg) |
|---|---|---|---|
| | | 1-5 | 1-5 |
| $t_{1/2}$ | h | 0.66±0.07 | 0.93±0.38 |
| $C_{0/}C_{max}$ | ng/mL | 2046.5±906.60 | 361.1±258.2 |
| $t_{max}$ | h | / | 0.35±0.14 |
| $AUC_{0-t}$ | ng.h/mL | 435.6±138.4 | 523.3±433.3 |
| $AUC_{0-\infty}$ | ng.h/mL | 438.3±138.8 | 658.9±370.6 |
| $MRT_{0-\infty}$ | h | 0.37±0.11 | 1.16±0.12 |
| F (bioavailability, %) | | - | 24.05 |

**5. Discussion**

After intragastric administration and intravenous administration of compound M2 and compound I-5 in rats, the results of pharmacokinetic studies showed that absorption of compound M2 and compound I-5 peaked quickly after intragastric administration, the peaking time $t_{max}$ was 0.35 h, $C_{max}$ of compound M2 was 425.4±136.9 ng/mL and $C_{max}$ of compound I-5 was 361.1±258.2 ng/mL, compound M2 had a high plasma concentration; $AUC_{0-t}$ of compound M2 was 1080.1±373.4 ng.h/mL and $AUC_{0-t}$ of compound I-5 was 523.3±433.3 ng.h/mL, and at the same intragastric administration dosage, compound M2 exhibited higher exposure in rat bodies and higher absolute bioavailability than

compound I-5, which were 40.8% and 24.05%, respectively.

**Experimental example 5: Study of anxiolytic effect of M2 and its structural analogues**

**I. Experimental materials**

**1. Tested samples**

As can be known according to the results of two experiments (mice elevated plus maze experiment and rat drinking conflict experiment) in the description of Patent C, compounds II-3, II-4, II-5, and II-10 had better effects in the two experiments, so the four compounds were selected as control to evaluate the anxiolytic effect of compound M2 in the present application.

① Name or code: Compound M2

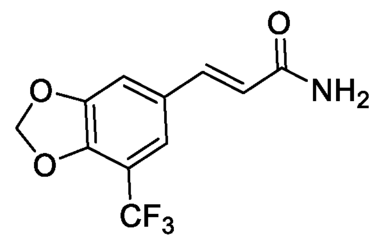

Batch No.: 20201029
Test product No.: YLS-2021-CMI1203-01-001
Source: Chemical Drugs Development Center of Tasly Research Institute

② Name or code: Compound II-3 (i.e. compound I-3 in Patents A and B)

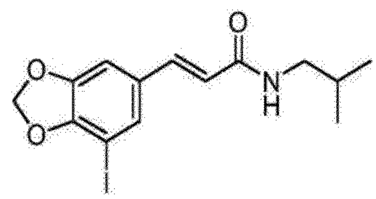

Batch No.: 20180521
Test product No.: YSL-2021-CMI1203-01-023
Source: Chemical Drugs Development Center of Tasly Research Institute

③ Name or code: Compound II-4 (i.e. compound I-4 in Patents A and B)

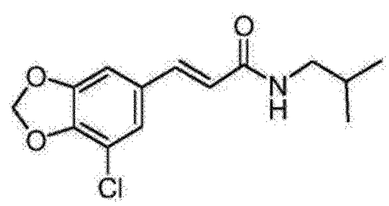

Batch No.: 20211217
Test product No.: YSL-2022-CMI1203-01-001
Source: Chemical Drugs Development Center of Tasly Research Institute

④ Name or code: Compound II-5 (i.e. compound I-5 in Patents A and B)

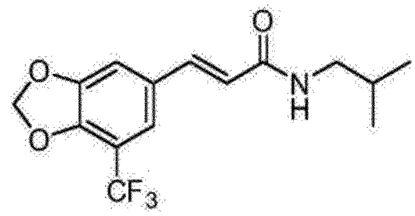

Batch No.: C16101006-C17001M
Test product No.: YSL-2021-CMI1203-01-002
Source: Chemical Drugs Development Center of Tasly Research Institute

⑤ Name or code: Compound II-10 (i.e. compound 1-10 in Patents A and B)

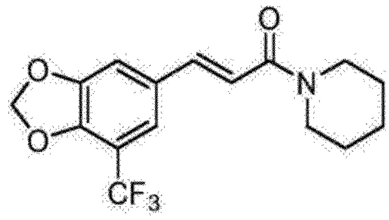

Batch No.: 20170601
Test product No.: YSL-2021-CMI1203-01-023
Source: Chemical Drugs Development Center of Tasly Research Institute

**2 Positive control products**

Name or code: Estazolam tablets

Batch No.: 211007

Test product No.: YSL-2021-CMI1203-01-022

Source: Shandong Xinyi Pharmaceutical Co., Ltd.

**3 Main instruments**

| Name of instrument | Instrument model | Manufacturer | Test items |
|---|---|---|---|
| Balance | T-1000 | Changshu G&G Measurement Plant | Weighing body weight |
| Electronic balance | MS204S | Mettler Toledo Instruments (Shanghai) Co., Ltd. | Weighing test products |
| Elevated plus maze | NA | Karwin, Nanjing | Anxiolytic test |
| Vogel conflict test system | LE100-25 | Panlab | Anxiolytic test |

## II. Experimental animals

### 2.1 Experimental animals

① Species: ICR mice (for elevated plus maze experiment)

Number: 120

Grade: SPF

Sex: Male

Weight: 18-20 g

Animal certificate No.: 110011211113772653, 110011221101333628

Source: Beijing Vital River Laboratory Animal Technology Co., Ltd.

Production license No.: SCXK (Beijing) 2021-0006

(2) Species: SD rats (for drinking conflict experiment)

Number: 75

Grade: SPF

Sex: Male

Weight: 180-200 g

Animal certificate No.: 110011221102490324

Source: Beijing Vital River Laboratory Animal Technology Co., Ltd.

Production license No.: SCXK (Beijing) 2021-0011

### 2.2 Animal facilities

Feeding facilities: within barrier environment of animal facilities of Tianjin Tasly Holding Group Co., Ltd.

Facility Address: within factory area at the intersection of Huaihe Road and Tingjiang West Road, Beichen Science and Technology Park, Tianjin

Laboratory animal use license: SYXK (Tianjin) 2017-0007

Issued by: Tianjin Science and Technology Commission

### 2.3 Animal feeding and management

Feeding environment: barrier environment, the environmental conditions of the facility met relevant standards on barrier animal experimental facilities in China's national standard "Laboratory animal - Requirements of environment and housing facilities" (GB14925-2001). Animal feeding management and animal experimental operations met the requirements of Regulations for the Administration of Affairs Concerning Experimental Animals in Tianjin and other regulations, with the temperature: 20-26°C, humidity: 40%-70%, lighting: 12 h bright and 12 h dark, ventilation: ≥15 times/h full fresh air, and animals drank sterile water (four-stage filtration and UV sterilization) prepared by 1T/h type multi-microporous membrane filtration system.

Animal management was the responsibility of the animal security department, which provided enough feed and drinking water for animals every day except fasting period. Drinking water bottles were changed once a day. Cushioning materials for animal feeding were changed twice a week, and at any time under special circumstances. Feeding cages were changed once a week; and animal feed: purchased from Beijing Keao Xieli Feed Co., Ltd., with the production license No.: SCXK (Beijing) 2019-0003.

### 2.4 Animal reception and quarantine

When the experimental animals arrived, they were received by the experiment personnel, veterinarians and the animal security department together. At the time of receiving, it was checked first whether the means of transport met the requirements, and then the animal certificate provided by the animal supply unit was checked to confirm whether the content of the certificate complied with the species, grade, number and sex of the animals for which purchase was applied. Then whether the outer package met the requirements and whether the outer package of animals was damaged were checked. The outer package of animals was passed into a quarantine room through a first transfer cabinet. Test equipment and test records were passed through a second transfer cabinet.
The outer package of animals was opened in the quarantine room, and whether the sex and number of animals were consistent with the items contained in the animal qualification certificate was checked. The animals were inspected for appearance (including sex, weight, head, trunk, tail, limbs, fur, mental state, activities, etc.) one by one, and the Reception Record of Experimental Animals and Quarantine Record of Experimental Animals were filled. After inspection, the animals were put into animal feeding cages, with tags of quarantine date hung on the cages, and then they were placed in the quarantine room for adaptation period feeding.
The adaptation feeding period of the animals was 2-3 days. The animals were observed regularly, including weight, head, trunk, tail, limbs, fur, mental state, activities, etc.

## III. Experimental method

### 3.1 Exploratory study on single administration dosage of compound M2 (elevated plus maze experiment)

(1) Animal grouping and drug administration

50 male ICR mice were randomly divided into 5 groups (a solvent control group, estazolam group, M2 high dosage group, M2 medium dosage group, M2 low dosage group), with 10 mice in each group. The compound M2 high, medium and low dosage groups were intragastrically administered at 20 mg/kg, 10 mg/kg and 5 mg/kg, respectively; the positive drug estazolam was intragastrically administered at 2.5 mg/kg; and the solvent control group was gavaged with an equal volume of solvent. Behavior test was performed in each group 30 min after single administration. The experiments were required to be conducted between 8:00 am and 14:00 pm, and all animals entered the test laboratory on the previous day.
Basis for dosage design: the results of previous anxiolytic experiment (Patent C) showed that the effective anxiolytic dosage of compounds II-3, II-4, and II-5, etc., structural analogues of compound M2, was 10 mg/kg in mice elevated plus maze experiment. In this experiment, with 10 mg/kg as the medium dosage of compound M2, a lower dosage of 5 mg/kg and a higher dosage of 20 mg/kg were tried respectively. The anxiolytic clinical dosage of the positive drug estazolam was 6 mg/person/day, which was converted into a clinical equivalent dosage of 6 mg/60kg*12.3=1.23 mg/kg for mice, with the double clinical dosage being 2.5 mg/kg; and was converted into a clinical equivalent dosage of 6 mg/60kg*6.2=0.62 mg/kg for rats, with the double clinical dosage being 1.24 mg/kg.

Table 9. Exploration of single administration dosage of compound M2-group and administration dosage

| Group | Dosage | Number of animals in each group | Mode of administration |
|---|---|---|---|
| Solvent control group | --- | 10 | single oral intragastric administration |
| Estazolam group | 2.5 mg/kg | 10 | |
| M2 high dosage group | 20 mg/kg | 10 | |
| M2 medium dosage group | 10 mg/kg | 10 | |
| M2 low dosage group | 5 mg/kg | 10 | |

(2) Behavior test

The laboratory was dimly lit (with the lowest brightness that can distinguish the subtle activities of mice at a distance of 1.5 m) and the brightness was kept constant, with a room temperature of about 20°C, and kept quiet. Before the maze test, each mouse was placed in a 35cm* 10cm*5cm plastic box and left to explore freely for 5 min and then quickly placed at the central platform of an elevated plus maze, with its head facing one of the open arms, and the following indexes were recorded upon release. Each mouse was tested for 5 min. Observers observed and recorded animal activities at a distance of 1.5 m. At intervals, the maze was wiped with a wet cloth to remove feces, and then wiped with a dry cloth before a next mouse was tested.

(3) Behavioral observation index

① The number of open arm entries (OE): the number of entries into any open arm, based on the fact that all four claws of the mouse entered the arm, and a complete exit of one claw from the arm indicated the completion of the entry activity;
② open arm time (OT): the time of entering an open arm, with the unit of seconds;
③ the number of close arm entries (CE): the number of entries into any close arm, based on the fact that all four claws of the mouse entered the arm;
① close arm time (CT): the time of entering a close arm, with the unit of seconds;
⑤ percentage of open arm time: OT%=OT/(OT+CT)* 100%; and
⑥ percentage of open arm entries: OE%=OE/(OE+CE)* 100%.

### 3.2 Comparative study on efficacy of compound M2 and its structural analogues in single administration (elevated plus maze experiment)

(1) Animal grouping and drug administration

70 male ICR mice were randomly divided into 7 groups (a solvent control group, estazolam group, compound M2 group, compound II-3 group, compound II-4 group, compound II-5 group, and compound II-10 group), with 10 mice in each group. The dosage of M2 and its four structurally similar compounds was determined as 20 mg/kg according to the experimental results of 3.1; the positive drug estazolam was administered intragastrically at 2.5 mg/kg; and the solvent control group was gavaged with an equal volume of solvent. Behavior test was performed in each group 30 min after single administration. The experiments were required to be conducted between 8:00 am and 14:00 pm, and all animals entered the test laboratory on the previous day.

Table 10. Single administration of compound M2 and structural analogues in elevated plus maze - group and dosage

| Group | Dosage | Number of animals in each group | Mode of administration |
|---|---|---|---|
| Solvent control group | --- | 10 | Single oral intragastric administration |
| Estazolam group | 2.5 mg/kg | 10 | |
| Compound M2 group | 20 mg/kg | 10 | |
| Compound II-3 group | 20 mg/kg | 10 | |
| Compound II-4 group | 20 mg/kg | 10 | |
| Compound II-5 group | 20 mg/kg | 10 | |
| Compound II-10 group | 20 mg/kg | 10 | |

The behavioral test method and behavioral observation indexes were the same as those in 3.1.

### 3.3 Comparative study on efficacy of compound M2 and structural analogues in single administration (drinking conflict experiment)

(1) Animal grouping and drug administration

75 male SD rats, with the body weight of 180-220 g, were fed adaptively for 1 week, water deprived for 24 h

for training period test. The animals screened to be qualified were randomly divided into 7 groups (a solvent control group, estazolam group, compound M2 group, compound II-3 group, compound II-4 group, compound II-5 group and compound II-10 group) according to their body weight, with 8 rats in each group. The dosage of compound M2 and its four structural analogues was determined as 10 mg/kg according to the experimental results of 3.1; the positive drug estazolam was administered intragastrically at 1.24 mg/kg; and the solvent control group was gavaged with an equal volume of solvent. The grouped animals were continued to be water deprived for 24 h and were given a single dose, and tested in a punishment experiment 30 min after the single dose.

Table 11. Drinking conflict of compound M2 and its structural analogues in single administration - group and dosage

| Group | Dosage | Number of animals in each group | Mode of administration |
|---|---|---|---|
| Solvent control group | --- | 8 | Single oral intragastric administration |
| Estazolam group | 1.24 mg/kg | 8 | |
| Compound M2 group | 10 mg/kg | 8 | |
| Compound II-3 group | 10 mg/kg | 8 | |
| Compound II-4 group | 10 mg/kg | 8 | |
| Compound II-5 group | 10 mg/kg | 8 | |
| Compound II-10 group | 10 mg/kg | 8 | |

The experiment was carried out in two stages. The first stage is a training stage, in which 24 h after water deprivation, animals were placed in an operation box separately, and were allowed to fully explore until they found the bottle mouth and started to lick water (with an intensity of electric shock being 0 mA), a counter automatically recorded the number of licks within 3 min, and the animals that licked water less than 300 times were eliminated. The second stage is a punishment stage, in which animals that were not eliminated continued to be water deprived for 24 h and then administered with drugs, and then placed in the operation box separately again, when the animals licked water for 20 times, the instrument automatically started timing and gave an electric shock (the ratio of licks to electric shocks was 20:1), the electric shock generally had an intensity of 0.3 mA and lasted for 2 s, but the animals could be released from the electric shock by disengaging from the bottle mouth, and the number of water licks and the number of electric shocks of the animals were recorded for 3 min.

Observation index: the number of water licks of rats in the punishment stage.

### 3.4 Statistical method

Data was expressed as mean±standard (mean±SD) difference, and statistics of data differences were made by one-way analysis of variance (ANOVA) or nonparametric test, and differences between groups were determined by $P<0.05$.

## IV. Experimental results

### 4.1 Exploratory study on single administration dosage of compound M2 (elevated plus maze experiment)

Results of the exploratory study on single administration dosage of compound M2 showed that high (20 mg/kg), medium (10 mg/kg), and low (5 mg/kg) dosages of compound M2 in single intragastric administration all could increase the number of open arm entries of mice and prolong the residence time of mice in the open arm in the elevated plus maze experiment to different degrees, with significant differences ($P<0.05$, $P<0.01$) between the compound M2 high dosage group and the solvent control group.

These results indicated that single administration of compound M2 had significant anxiolytic activity, and the effective dosage of compound M2 for single administration was 20 mg/kg.

Table 12. Effects of different dosages of compound M2 on the number of open arm entries and the residence time in open arm of mice

| Group | Dosage | OE% | OT% |
|---|---|---|---|
| Solvent control group | --- | 29.36±10.96 | 26.53±13.42 |
| Estazolam group | 2.5 mg/kg | 42.78±15.27* | 50.12±12.76** |

(continued)

| Group | Dosage | OE% | OT% |
|---|---|---|---|
| Compound M2 high dosage group | 20 mg/kg | 38.47±7.13* | 44.60±9.33** |
| Compound M2 medium dosage group | 10 mg/kg | 36.13±14.12 | 35.92±13.11 |
| Compound M2 low dosage group | 5 mg/kgn | 35.46±10.13 | 38.52±12.19 |
| Note: Compared with the solvent control group, *P<0.05, **P<0.01 | | | |

**4.2 Comparative study on efficacy of compound M2 and its structural analogues in single administration (elevated plus maze experiment)**

The study results of the elevated plus maze experiment showed that compound M2 and its structural analogues II-3, II-4, II-5 and II-10 at single intragastric administration of 20 mg/kg all could increase the number of open arm entries of mice and prolong the residence time of mice in open arm in the elevated plus maze experiment to different degrees, wherein compound M2, compound II-4, compound II-5 and compound II-10 groups had significant differences (P<0.05, P<0.01) compared with the solvent control group, which indicated that compound M2 and its structural analogues II-4, II-5 and II-10 had significant anxiolytic activity in the model, and compound M2 had the best anxiolytic activity compared with II-3, II-4, II-5 and II-10.

Table 13. Effects of compound M2 and its structural analogues on the number of open arm entries and the residence time in open arm of mice

| Group | Dosage | OE% | OT% |
|---|---|---|---|
| Solvent control group | --- | 33.03±9.45 | 30.52±11.47 |
| Estazolam group | 2.5 mg/kg | 69.60±15.12** | 73.46±20.29** |
| Compound M2 group | 20 mg/kg | 57.94±11.72** | 57.36±8.77** |
| Compound II-3 group | 20 mg/kg | 41.04±9.52## | 42.99±11.82## |
| Compound II-4 group | 20 mg/kg | 46.79±9.31**# | 53.45±8.33** |
| Compound II-5 group | 20 mg/kg | 47.01±6.30**# | 46.70±9.98*# |
| Compound II-10 group | 20 mg/kg | 50.00±10.23** | 52.08±12.25** |
| Note: Compared with the solvent control group, *P<0.05, **P<0.01; and compared with M2, #P<0.05, ##P<0.01 | | | |

**4.3 Comparative study on efficacy of compound M2 and its structural analogues in single administration (drinking conflict experiment)**

The study results of drinking conflict experiment showed that compound M2 and its structural analogues II-3, II-4, II-5 and II-10, at single intragastric administration of 20 mg/kg, all could increase, to different extents, the drinking times of rats in the drinking conflict experiment, wherein compound M2, compound II-5 and compound II-10 groups had significant differences (P<0.05) compared with the solvent control group, which indicated that compound M2 and its structural analogues II-5 and II-10 had significant anxiolytic activity in the model, and compound M2 had the best anxiolytic activity compared with II-3, II-4, II-5 and II-10.

Table 14. Effects of compound M2 and its structural analogues on the number of times of drinking water of Vogel drinking conflict model rats

| Group | Dosage | Number of animals in each group | Number of times of drinking water |
|---|---|---|---|
| Solvent control group | --- | 8 | 306.94±203.83 |
| Estazolam group | 1.24 mg/kg | 8 | 540.41/kg2.25* |
| Compound M2 group | 10 mg/kg | 8 | 527.26±143.66* |
| Compound II-3 group | 10 mg/kg | 8 | 363.96±145.26# |

(continued)

| Group | Dosage | Number of animals in each group | Number of times of drinking water |
|---|---|---|---|
| Compound II-4 group | 10 mg/kg | 8 | 387.07±57.31# |
| Compound II-5 group | 10 mg/kg | 8 | 478.02±106.32* |
| Compound II-10 group | 10 mg/kg | 8 | 499.61±133.93* |
| Note: Compared with the solvent control group, *P<0.05; and compared with M2, #P<0.05 | | | |

**V. Experimental conclusions**

To sum up, compound M2 had significant anxiolytic activity, and the effective dosage of single administration was 20 mg/kg (mice). Compared with its structural analogues II-3, II-4, II-5 and II-10, compound M2 had better anxiolytic activity.

## Claims

1. A substituted cinnamamide compound, having the following structural formula:

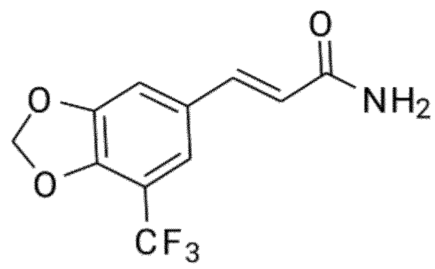

M2.

2. An antidepressant and anxiolytic compound, having the following structural formula:

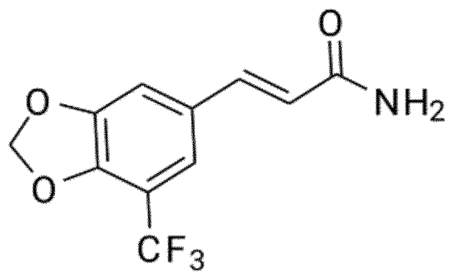

M2.

3. The compound according to claim 1 or 2, wherein the compound can also be present in a form of a solvate.

4. The compound according to claim 1 or 2, wherein the compound can also be present in a form of a pharmaceutically acceptable salt.

5. A pharmaceutical composition comprising the compound according to any one of claims 1-4.

6. The pharmaceutical composition according to claim 5, wherein the pharmaceutical composition can be in any doseable pharmaceutical form; optionally, the pharmaceutical form is selected from: tablets, capsules, oral liquids, oral lozenge, granules, pills, pulvises, pastes, pellets, suspensions, powders, solutions, injections, suppositories, soft ointments, hard ointments, creams, sprays, drops and patches; the tablets are optionally sugar-coated tablets, film-coated tablets or enteric-coated tablets; the capsules are optionally hard capsules or soft capsules; and the injections are optionally one of injection fluids, freeze-dried powder injections and liquid drug injections.

**7.** The pharmaceutical composition according to claim 5, wherein when in use, the pharmaceutical composition can be used in combination with other antidepressant drugs or anxiolytic drugs, or the pharmaceutical composition further comprises another antidepressant drug or anxiolytic drug; the other antidepressant drug or anxiolytic drug being selected from: nefazodone, sulpiride, alprazolam, lorazepam, buspirone, tandospirone, methylphenidate, fluoxetine, paroxetine, sertraline, citalopram, lexapro, fluvoxamine, reboxetine, venlafaxine, flupenthixol, melitracen, and Neurostan.

**8.** A preparation method of the compound according to claim 1 or 2, wherein the preparation method being:

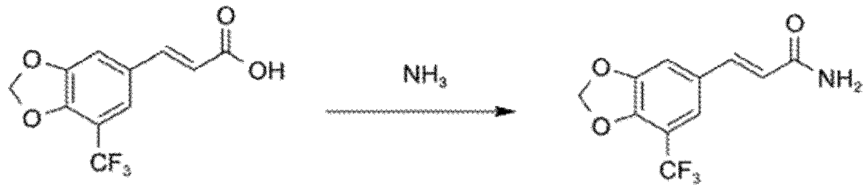


Intermediate A M2

reacting (E)-3-(3',4'-methylenedioxy-5'-trifluoromethyl-phenyl)-acrylic acid (i.e. intermediate A) with $NH_3$ to give compound M2.

**9.** The preparation method of the compound according to claim 8, comprising the following reaction route:

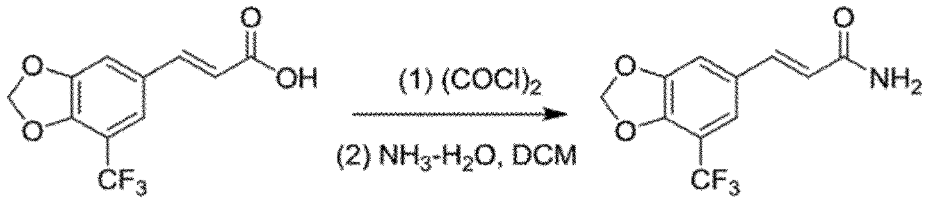


Intermediate A M2

**10.** The preparation method according to claim 9, comprising the following operation steps:
dissolving (E)-3-(3',4'-methylenedioxy-5'-trifluoromethyl-phenyl)-acrylic acid (i.e. intermediate A) in dichloromethane, adding a catalytic amount of N,N-dimethylformamide, dropping oxalyl chloride under ice bath, stirring at room temperature until the completion of reaction of raw materials; concentrating and drying the reaction liquid, then adding dichloromethane to dissolve, dropping ammonia water under ice bath, reacting at room temperature until the completion of the reaction; and removing the solvent by evaporation under reduced pressure, then adding dilute hydrochloric acid to adjust it to be acidic, precipitating a solid, filtering and rinsing it with water to obtain a crude product, which is then subjected to silica gel column chromatography for purification to give M2.

**11.** Use of the compound according to any one of claims 1-4 or the pharmaceutical composition according to any one of claims 5-7 in a medicine.

**12.** Use of the compound according to any one of claims 1-4 or the pharmaceutical composition according to any one of claims 5-7 in the preparation of an antidepressant, anxiolytic, or antidepressant and anxiolytic drug.

**13.** Use of the compound according to any one of claims 1-4 or the pharmaceutical composition according to any one of claims 5-7 in combination with another antidepressant drug or anxiolytic drug, or use of the compound according to any one of claims 1-4 in combination with one or more additional antidepressant drugs or anxiolytic drugs in the

preparation of an antidepressant, anxiolytic, or antidepressants and anxiolytic drug; here, the other antidepressant drugs or anxiolytic drugs, or the additional antidepressant drugs or anxiolytic drugs can be selected from one or more of the following drugs for prevention and treatment of psychiatric disorders: e.g., nefazodone, sulpiride, alprazolam, lorazepam, buspirone, tandospirone, methylphenidate, fluoxetine, paroxetine, sertraline, citalopram, lexapro, fluvoxamine, reboxetine, venlafaxine, flupenthixol, melitracen, and Neurostan.

**14.** A method for preventing or treating psychiatric disorders, comprising administering the compound according to any one of claims 1-4 or the pharmaceutical composition according to any one of claims 5-7 to a patient in need thereof.

**15.** The method according to claim 14, wherein the preventing or treating psychiatric disorders is anti-depression, anti-anxiety or anti-depression and anti-anxiety.

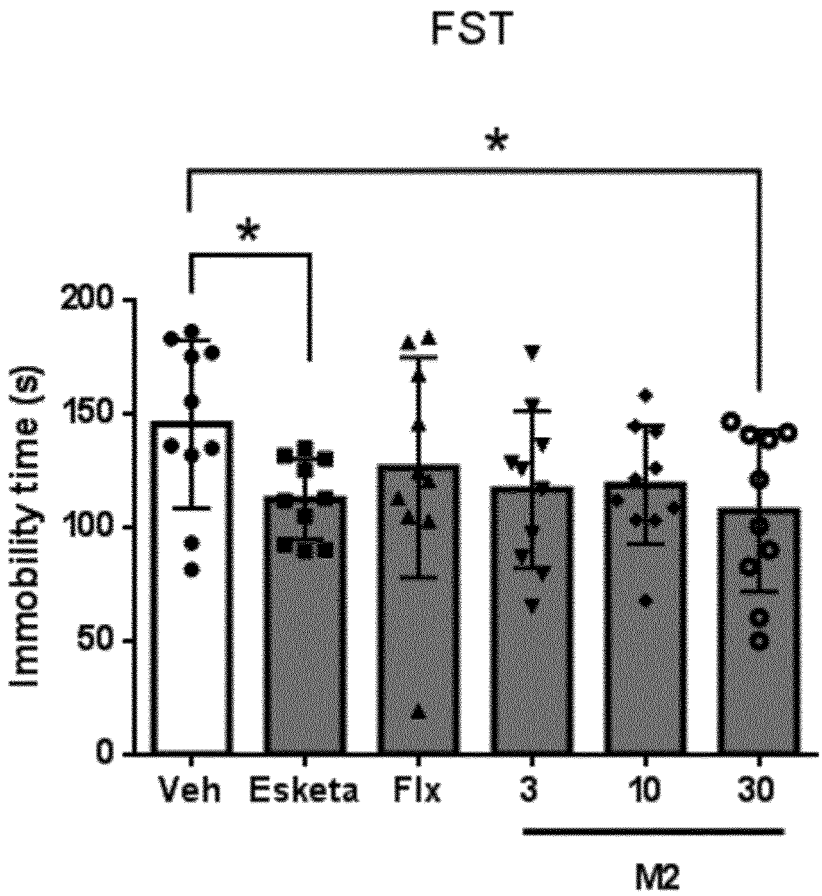


FIG. 1

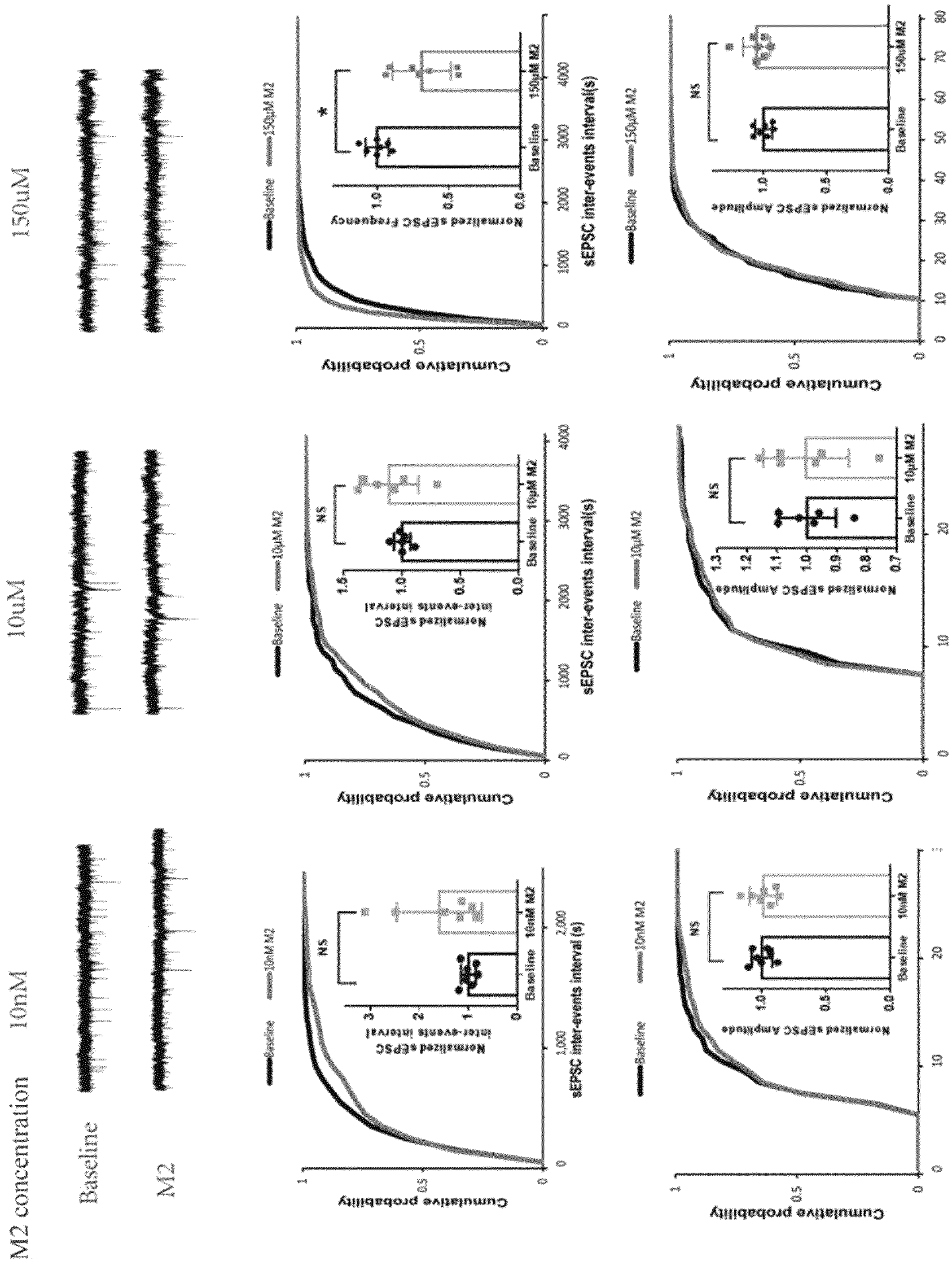
M2 concentration
10nM
10uM
150uM
Baseline
M2
Cumulative probability
sEPSC inter-events interval (s)
sEPSC inter-events interval(s)
sEPSC Amplitude(pA)
Normalized sEPSC inter-events interval
Normalized sEPSC Frequency
Normalized sEPSC Amplitude
Baseline
10nM M2
10μM M2
150μM M2
NS
*

FIG. 2

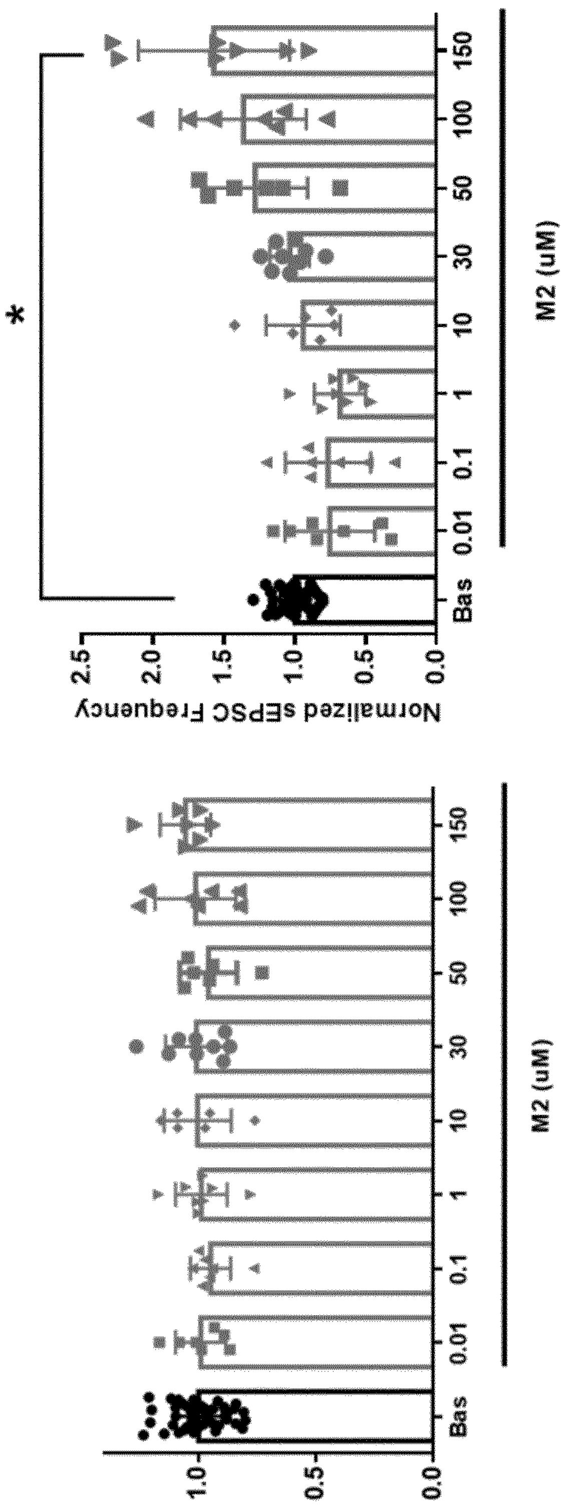
Normalized sEPSC Amplitude
1.0
0.5
0.0
Bas
0.01
0.1
1
10
30
50
100
150
M2 (uM)
Normalized sEPSC Frequency
2.5
2.0
1.5
1.0
0.5
0.0
*
Bas
0.01
0.1
1
10
30
50
100
150
M2 (uM)

FIG. 3

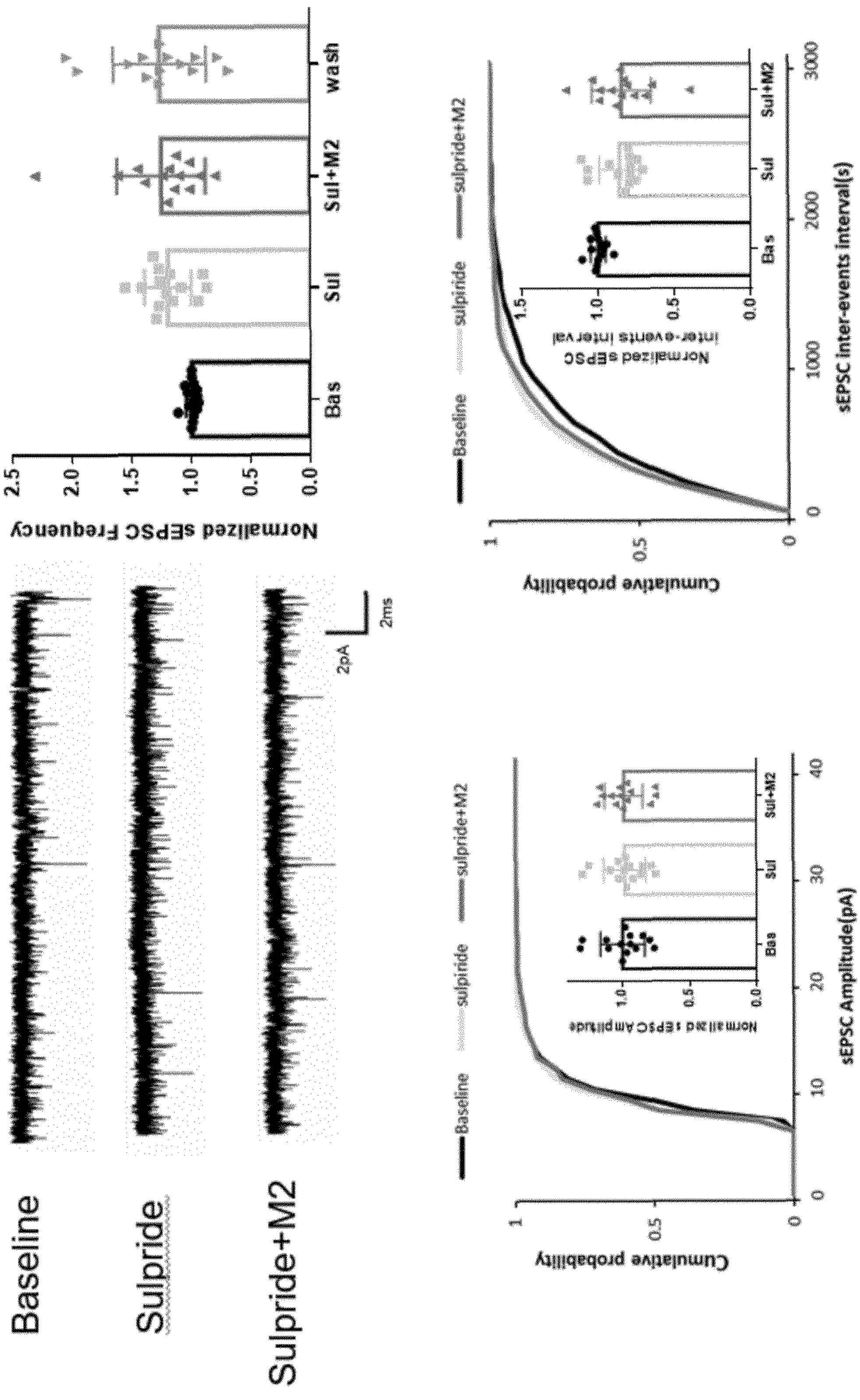
Baseline
Sulpride
Sulpride+M2
2pA
2ms
Normalized sEPSC Frequency
2.5
2.0
1.5
1.0
0.5
0.0
Bas
Sul
Sul+M2
wash
Baseline
sulpride
sulpride+M2
Cumulative probability
sEPSC Amplitude(pA)
Normalized sEPSC Amplitude
sEPSC inter-events interval(s)
Normalized sEPSC inter-events interval

FIG. 4

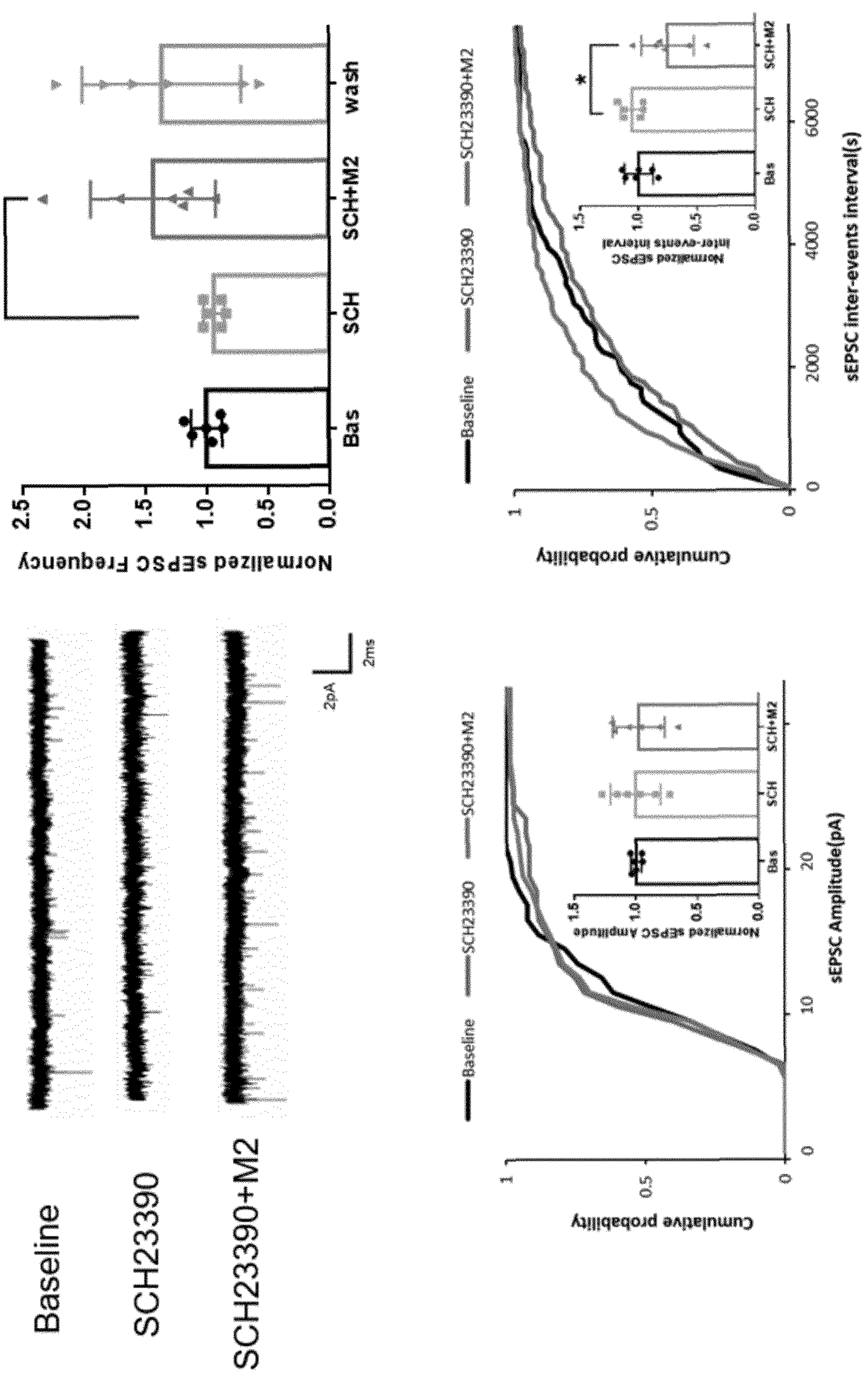
Baseline
SCH23390
SCH23390+M2
2pA
2ms
Normalized sEPSC Frequency
Bas
SCH
SCH+M2
wash
*
Cumulative probability
sEPSC Amplitude(pA)
Normalized sEPSC Amplitude
sEPSC inter-events interval(s)
Normalized sEPSC inter-events interval

FIG. 5

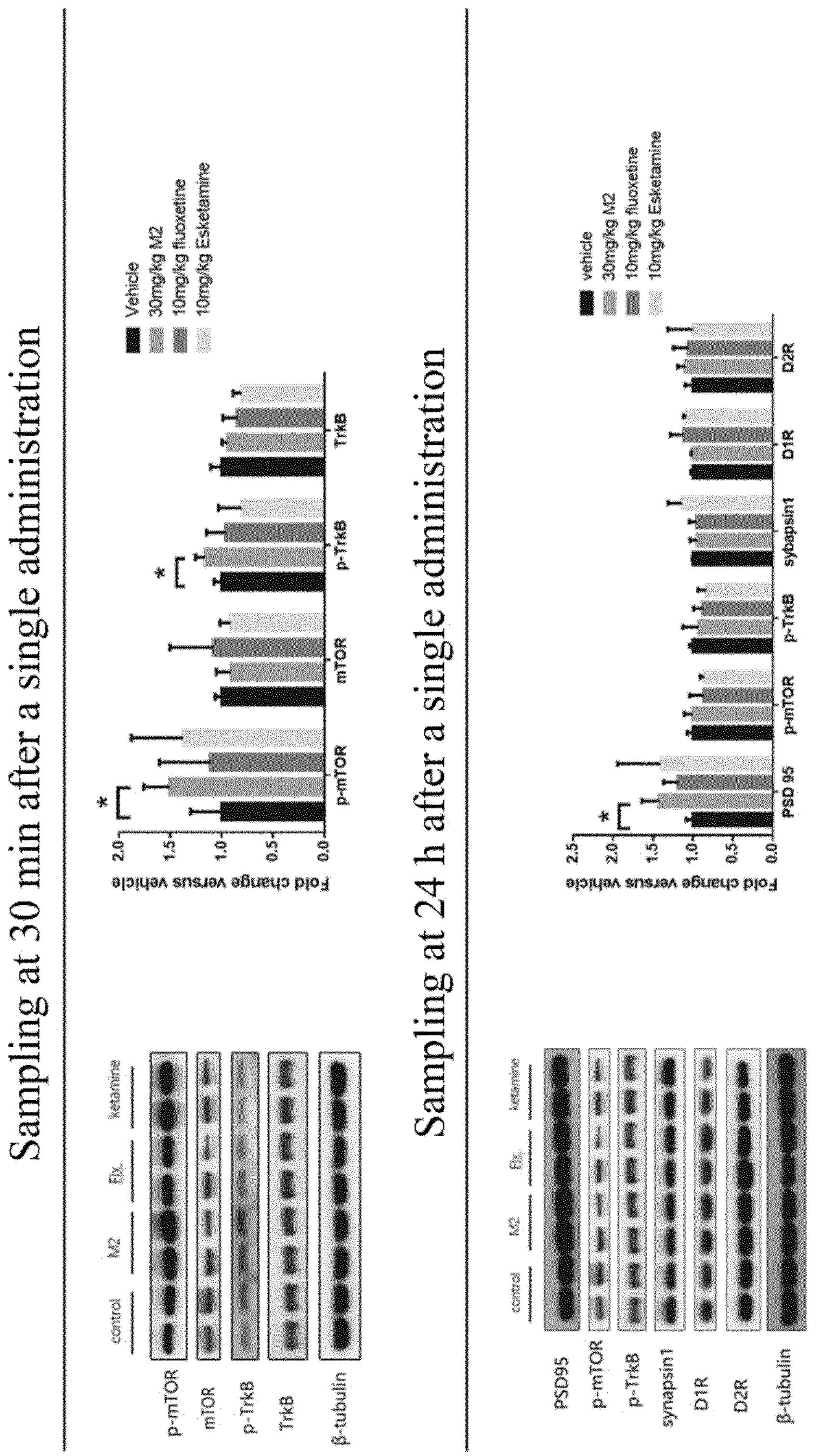
a
Sampling at 30 min after a single administration
p-mTOR
mTOR
p-TrkB
TrkB
β-tubulin
control
M2
Flx
ketamine
Fold change versus vehicle
Vehicle
30mg/kg M2
10mg/kg fluoxetine
10mg/kg Esketamine
b
Sampling at 24 h after a single administration
PSD95
synapsin1
D1R
D2R
vehicle
sybapsin1
PSD 95

FIG. 6

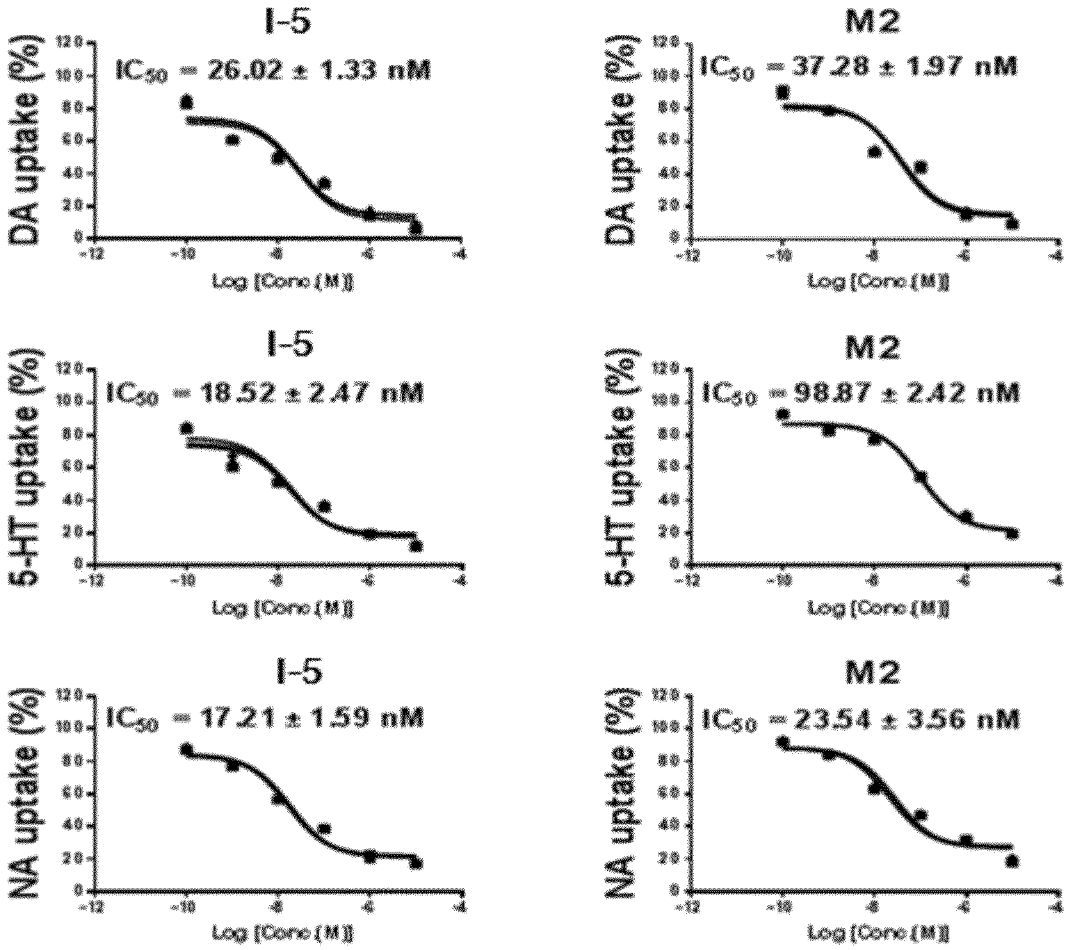
I-5
DA uptake (%)
IC50 = 26.02 ± 1.33 nM
Log [Conc (M)]
M2
DA uptake (%)
IC50 = 37.28 ± 1.97 nM
Log [Conc (M)]
I-5
5-HT uptake (%)
IC50 = 18.52 ± 2.47 nM
Log [Conc (M)]
M2
5-HT uptake (%)
IC50 = 98.87 ± 2.42 nM
Log [Conc (M)]
I-5
NA uptake (%)
IC50 = 17.21 ± 1.59 nM
Log [Conc (M)]
M2
NA uptake (%)
IC50 = 23.54 ± 3.56 nM
Log [Conc (M)]

FIG. 7

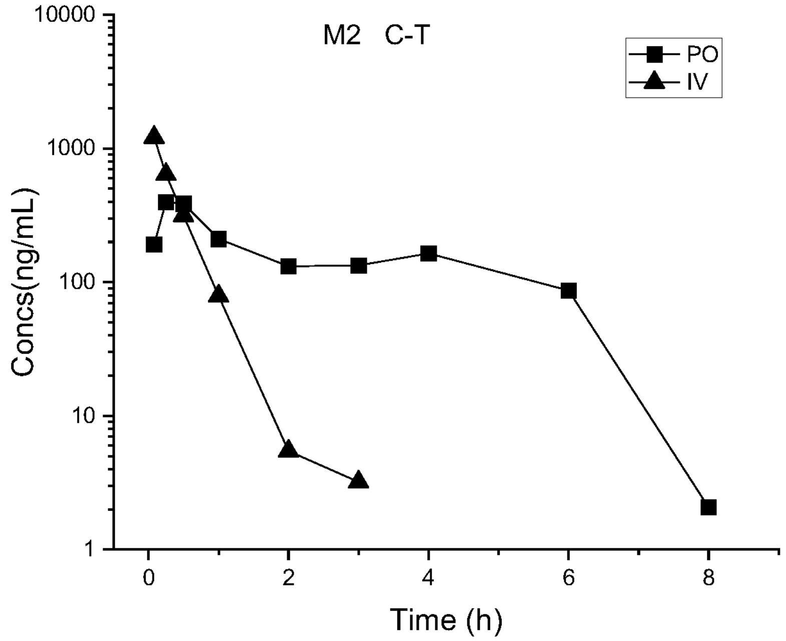
M2 C-T
PO
IV
Concs(ng/mL)
Time (h)
10000
1000
100
10
1
0
2
4
6
8

FIG. 8

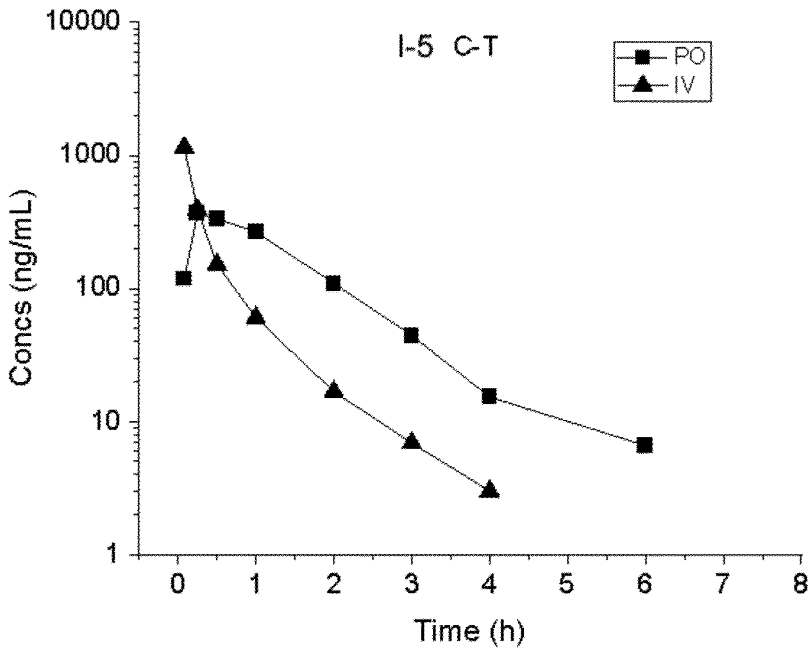
I-5 C-T
PO
IV
Concs (ng/mL)
Time (h)
10000
1000
100
10
1
0
1
2
3
4
5
6
7
8

FIG. 9

**INTERNATIONAL SEARCH REPORT**

International application No. **PCT/CN2022/094753**

**A. CLASSIFICATION OF SUBJECT MATTER**

C07D 317/50(2006.01)i; A61K 31/36(2006.01)i; A61P 25/24(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

CNPAT; WPI; Web of science; REGISTRY(STN); CAPLUS(STN); CASlink(STN): 天士力, 桂皮酰胺, 焦虑, 抑郁, crnnamamide, epressive, psychoses

**C. DOCUMENTS CONSIDERED TO BE RELEVANT**

| Category* | Citation of document, with indication, where appropriate, of the relevant passages | Relevant to claim No. |
|---|---|---|
| X | CN 103687850 A (TASLY PHARMACEUTICAL GROUP CO., LTD.) 26 March 2014 (2014-03-26)<br>abstract, claim 1, and description, pp. 3-7 | 1-15 |
| A | CN 102850317 A (TASLY PHARMACEUTICAL GROUP CO., LTD.) 02 January 2013 (2013-01-02)<br>abstract, and description, p. 3 | 1-15 |
| A | CN 105708797 A (TASLY PHARMACEUTICAL GROUP CO., LTD.) 29 June 2016 (2016-06-29)<br>claim 3 | 1-15 |
| A | CN 102558133 A (63975 TROOPS OF THE PEOPLE'S LIBERATION ARMY) 11 July 2012 (2012-07-11)<br>claims 1-7 | 1-15 |
| A | CN 107011313 A (TASLY PHARMACEUTICAL GROUP CO., LTD.) 04 August 2017 (2017-08-04)<br>claim 1 | 1-15 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 August 2022** | **23 August 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/094753**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14-15**
because they relate to subject matter not required to be searched by this Authority, namely:

[1] The technical solutions of claims 14-15 comprise a method for treating diseases as defined in PCT Rule 39.1(iv). The present search report was made on the basis of the technical solution of a corresponding use in the preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/094753**

| Patent document cited in search report | | | Publication date (day/month/year) | Patent family member(s) | | | Publication date (day/month/year) |
|---|---|---|---|---|---|---|---|
| CN | 103687850 | A | 26 March 2014 | US | 2014121242 | A1 | 01 May 2014 |
| | | | | KR | 20170082641 | A | 14 July 2017 |
| | | | | CN | 102850317 | A | 02 January 2013 |
| | | | | KR | 20140036294 | A | 25 March 2014 |
| | | | | AU | 2012276114 | A1 | 09 January 2014 |
| | | | | JP | 2014523886 | A | 18 September 2014 |
| | | | | WO | 2013000399 | A1 | 03 January 2013 |
| | | | | EP | 2725018 | A1 | 30 April 2014 |
| | | | | TW | 201309666 | A | 01 March 2013 |
| | | | | RU | 2014102302 | A | 10 August 2015 |
| CN | 102850317 | A | 02 January 2013 | TW | 201309666 | A | 01 March 2013 |
| | | | | CN | 103687850 | A | 26 March 2014 |
| | | | | CA | 2838495 | A1 | 03 January 2013 |
| | | | | US | 2014121242 | A1 | 01 May 2014 |
| | | | | KR | 20170082641 | A | 14 July 2017 |
| | | | | KR | 20140036294 | A | 25 March 2014 |
| | | | | AU | 2012276114 | A1 | 09 January 2014 |
| | | | | JP | 2014523886 | A | 18 September 2014 |
| | | | | WO | 2013000399 | A1 | 03 January 2013 |
| | | | | EP | 2725018 | A1 | 30 April 2014 |
| CN | 105708797 | A | 29 June 2016 | | None | | |
| CN | 102558133 | A | 11 July 2012 | | None | | |
| CN | 107011313 | A | 04 August 2017 | AU | 2017213007 | A1 | 07 June 2018 |
| | | | | WO | 2017129061 | A1 | 03 August 2017 |
| | | | | RU | 2018122135 | A | 23 December 2019 |
| | | | | US | 2019046500 | A1 | 14 February 2019 |
| | | | | IL | 260068 | A | 31 July 2018 |
| | | | | JP | 2019503368 | A | 07 February 2019 |
| | | | | CA | 3007870 | A1 | 03 August 2017 |
| | | | | KR | 20180103846 | A | 19 September 2018 |
| | | | | EP | 3409664 | A1 | 05 December 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202110606431
- CN 102850317 A
- WO 201210123842 A
- CN 103687850 A
- WO 201280020049 A
- CN 107011313 A
- WO 201710038281 A
- GB 149252001 A

### Non-patent literature cited in the description


- **SHINOHARA R ; AGHAJANIAN G K ; ABDALLAH C G.** Neurobiology of the Rapid Acting Antidepressant Effects of Ketamine: Impact and Opportunities[J. *Biological Psychiatry,* 2020
- **PIZZAGALLI D A ; ROBERTS A C.** Correction: Prefrontal cortex and depression[J. *Neuropsychopharmacology,* 2021, 1-1